# EUROPEAN PATENT SPECIFICATION

(11) **EP 4 490 233 B1**
(45) Date of publication and mention of the grant of the patent: **21.05.2025**
(21) Application number: 23708519.6
(22) Date of filing: 07.03.2023
(51) Int. Cl.: C08L 5/10, C08L 89/00, C08L 99/00

(54) **HEPARIN AND MIXTURES OF NATIVE PROTEINS AND PEPTIDES FROM WASTE TISSUE OF SLAUGHTERED ANIMALS**
HEPARIN UND MISCHUNGEN VON NATIVEN PROTEINEN UND PEPTIDEN AUS ABFALLGEWEBE VON SCHLACHTTIEREN
HÉPARINE ET MÉLANGES DE PROTÉINES ET DE PEPTIDES NATIFS PROVENANT DE TISSUS RÉSIDUELS D'ANIMAUX ABATTUS

(30) Priority: 08.03.2022 EP 22382214
(43) Date of publication of application: 15.01.2025
(73) Proprietor: Horizon IP, S.L., 17003 Girona (ES)
(72) Inventor: ESCAICH FERRER, José, 08021 BARCELONA (ES); RAMIREZ RUBIO, Pablo, 17300 BLANES (ES); CARLINO, Stefano, CH1868 COLLOMBEY (CH); CHETRIT RUSSO, Carlos, 17820 BANYOLES (ES); SALVATELLA SUREDA, Pau, 17240 LLAGOSTERA (ES); DALMAU PUIG, Pilar, 17118 SANT SANDURNÍ DE L'HEURA (ES); PUIGSEGUR SARABIA, Judith, 17220 SANT FELIU DE GUÍXOLS (ES); MONTERO PARDILLO, Marc, 17180 VILABLAREIX (ES)
(74) Representative: ZBM Patents - Zea, Barlocci & Markvardsen
(86) International application number: PCT/EP2023/055750
(87) International publication number: WO 2023/170064

(56) References cited:
- WO-A1-2020/163926
- CN-A- 102 839 162
- CONRAD ME ET AL: "Newly identified iron-binding protein in human duodenal mucosa", BLOOD, vol. 79, no. 1, 1 January 1992 (1992-01-01), pages 244 - 247, XP055953073, Retrieved from the Internet <URL:AQECAHi208BE49Ooan9kkhW_Ercy7Dm3ZL_9Cf3qfKAc485ysgAAA80wggPJBgkqhkiG9w0BBwagggO6MIIDtgIBADCCA68GCSqGSIb3DQEHATAeBglghkgBZQMEAS4wEQQMGy> [retrieved on 20220819], DOI: 10.1182/blood.V79.1.244.bloodjournal791244

## Description

This application claims the benefit of European Patent Application 22382214.9 filed March 8th, 2022.

### Technical Field

Present invention relates to the field of obtention of heparin, proteins and peptides in their native form, and other compounds of interest, from slaughtered animals for consumption of meat.

### Background Art

In the processing of the several parts of slaughtered animals, mainly for food consumption, besides the parts used as food, remaining extracted viscera not commonly offered to meat consumers are further processed in order to obtain several products of interest for other fields. For example, it is widely known the method of obtention of heparin of high pure grade and useful in medicine from the mucosal tissue, in particular from the intestine mucosa. After the recovery of the mucosa, intestine tubular structures are mainly recycled for many different purposes, including the casing in sausage elaboration or even in medical applications such as implants.

In these processes for extracting heparin, for example, aggressive mechanical homogenization of mucosal tissues (i.e., intestine) is carried out at high temperatures (60°C-80°C or even higher temperatures) in the presence of proteolytic enzymes, and/or by means of salting out the homogenates. Next, precipitation steps, optional extractive steps, and chromatography are carried out. These methodologies give from the one side the heparin, in several purity grades depending on the steps of the methods, and from the other side, a protein hydrolysate, considered a by-product and which is commonly used in animal food as peptide supplementation. Examples of documents disclosing method of extraction of heparin and other by products from small intestinal mucosa include Chinese patent application CN107236059 (Shenqing) and the Chinese patent CN106035980B (University of Jiangnan).

Proteins in their natural state with intact structure and function that is not altered by heat, chemicals, enzyme reaction, or other denaturants are named "native proteins". To date, in these methods of extraction of heparin, no proteins or peptides in native form are obtained, because high temperatures, enzymatic treatment, and mechanical stress reduce the same to the peptide hydrolysate. This hydrolysate is mostly made up of very small peptides and free amino acids not of high value. On the other hand, the regulations for the obtaining of heparin with the adequate purity grade and requirements imposed by health authorities for the medical application, do not allow many variations to the methodology for the obtaining of said compound.

Active enzymes and mixtures of active enzymes have also been obtained from mucosal tissues of slaughtered animals though. As a way of example, Chinese patent CN102839162B (Tibet Liyang technology Co Ltd) discloses a method to obtain alkaline phosphatase from animal viscera (including pig intestine mucosa), in which mucosa is first cut and homogenized at 4 °C; then it is precipitated with acid, extracted with butanol and dialyzed; further it is salted out with ammonium sulphate; and finally, the salted-out fraction is submitted to several chromatography columns to be further purified. An alkaline phosphatase product with a specific activity of 415 U/mg is so obtained. This method, however, does not allow the option of obtaining heparin or other enzymes according to prescribed regulations.

Proteolytic enzymes are obtained from fish digestive organs, for example following the method disclosed in Russian patent RU2610669. In this case, tissues are homogenized, filtered, and proteins are salted out in ammonium sulphate with a previous step of nucleic acids and lipids removal. Further, the precipitate is dissolved in a buffer and chromatography is performed.

All these methods to recover one or more enzymes in active form from animal mucosal tissues include a sequence of steps conceived to preserve enzyme integrity. They are performed at low and middle temperatures (4°C-37°C), and they are carried out at physiological conditions (of pH and ionic force).

In all the methods disclosed above, either for extraction of heparin or for the obtention of active enzymes, the main aim is to recover the product of interest in the balance of a high yield, activity and high purity.

Besides these methods departing from tissue of slaughtered animals, many enzymes are currently obtained using biotechnological methods including the expression of the enzymes in bioreactors. In these biotechnological methods, host cells (yeast, bacteria, etc.) are modified by means of recombinant gene technology to express the enzymes of interest when cultured. However, these methods are usually expensive, and they require of many reagents and energy suppliers to control growing conditions of the cells. Thus, they are usually considered methods of certain complexity.

Enzymes are used in several field of industry, as well as in the medical field. For example, cellulases and ligninases are used in biofuel industry, nucleases in the field of molecular biology, amylases and proteases in food processing, or xylanases in paper industry. Many enzymes are also used in clinical diagnostic methods. In the field of food supplementation and additivation of animal food, phytases have been employed to take profit of phosphorus of phytic acid usually contained in the mixture constituting fodders.

Albeit the existing methods, there is still a need of alternative methodologies to give value to products of slaughtered animals, obtained in the course of reproducible, non-complex and non-expensive processes, and allowing obtention of as much as possible reliable and effective by-products in high yields and complying with the regulations, if any. In the particular case of enzymes, there still remains a need in the field of additivation of food of active enzymes that can be obtained by reliable and economic processes.

### Summary of Invention

With the aim of giving value to as many products as possible from discarded tissues of slaughtered animals, inventors propose a new method allowing obtaining simultaneously heparin and proteins and peptides in their native form, of mammalian intestine mucosa by a non-expensive but sustainable approach. Heparin is obtained with a grade useful for pharmaceutical application (i.e., at pharmaceutical grade according to regulations). Moreover, the proteins and peptides are obtained in their native state, thus, they are in their properly folded and assembled form with operative structure and function (e.g., enzymatic, structural, hormonal, etc).

Present invention is, thus, embedded in the context of the processing of slaughterhouse tissues, from which value-added products are obtained after tissue homogenization, such as heparin.

The new methodology includes homogenization of mammal tissue at mild conditions (cold temperature and physiological pH), which helps preserving stability and activity of isolated proteins and peptides. Several additional steps including fractionation steps and chromatography of homogenate material are also performed.

Thus, a first aspect of the invention is a method for the simultaneous obtention of heparin, proteins and peptides in native state by fractionation of mammalian intestine mucosa, comprising the following steps:
(i) adding to an aqueous extract of mammalian intestinal mucosa a preservative and antioxidant agent to obtain a preserved mucosa, or preserving the aqueous extract at a temperature allowing preserving proteins, peptides and heparin in native state;
(ii) Optionally diluting the preserved mucosa with up to 100 volumes of deionized water, to obtain a diluted mucosa.
(iii) submitting diluted mucosa of step (ii) or preserved mucosa of (i) to homogenization at a temperature of less than 40 °C, to lyse cell membrane of mucosa cells to obtain a stable homogenate of the mucosa comprising heparin and mucosa proteins and peptides;
(iv) separating heparin and the mucosa proteins and peptides contained in the stable homogenate obtained in step (iii) by one or more of physical and/or chemical means selected from the group consisting of centrifugation, filtration and/or ultrafiltration, optionally using detergents, and combinations thereof, to obtain a pellet fraction comprising heparin (P) and proteins; and a supernatant fraction (SN) comprising mucosa proteins and peptides;
(v.a) submitting the pellet fraction (P) of (iv) comprising heparin to an alkaline proteolytic process by means of proteolytic enzymes to obtain a mixture of heparin and a protein hydrolysate, submiting the mixture with heparin to one or more precipitation steps, and optionally to one or more of extractive steps, and to one or more of a chromatography to purify the said heparin from the protein hydrolysate; and
(v.b) submitting the supernatant (SN) of (iv) comprising a mixture of solubilized and/or suspended mucosa proteins and peptides to one or more of successive filtration, ultrafiltration steps, protein precipitation and/or gel permeation and/or to ion exchange chromatography steps, being exchangeable the order or sequence of the steps, in order to separate the proteins and peptides according to any of their solubility, isoelectric point and/or molecular weight.

Disclosed is, therefore, a method for the simultaneous obtention of heparin, proteins and peptides in native state by fractionation of mammalian intestine mucosa, comprising the following steps:
(i) adding to an aqueous extract of mammalian intestinal mucosa a preservative and antioxidant agent to obtain a preserved mucosa, or preserving the aqueous extract at a temperature allowing preserving proteins, peptides and heparin in native state;
(ii) Optionally diluting the preserved mucosa with up to 100 volumes of deionized water, to obtain a diluted mucosa;
(iii) submitting diluted mucosa of step (ii) or preserved mucosa of (i) to homogenization at a temperature of less than 25 °C, to lyse cell membrane of mucosa cells to obtain a stable homogenate of the mucosa comprising heparin and mucosa proteins and peptides; and
(iv) generically separating heparin and the mucosa proteins and peptides contained in the stable homogenate obtained in step (iii) by one or more of physical and/or chemical means selected from the group consisting of centrifugation, filtration and/or ultrafiltration, optionally using detergents, and combinations thereof, to obtain a pellet fraction comprising heparin (P) and proteins; and a supernatant fraction (SN) comprising mucosa proteins and peptides.

Another aspect of the invention is a method for the simultaneous obtention of heparin, proteins and peptides in native state by fractionation of mammalian intestine mucosa, comprising the following steps:
(i) adding to an aqueous extract of mammalian intestinal mucosa a preservative and antioxidant agent to obtain a preserved mucosa, or preserving the extract at a temperature allowing preserving proteins, peptides and heparin in native state;
(ii) optionally diluting the preserved mucosa with up to 100 volumes of deionized water, to obtain a diluted mucosa
(iiia) submitting diluted mucosa of step (ii) or preserved mucosa of (i) to homogenization at a temperature of less than 40 °C, optionally in presence of detergents and/or hydrolytic enzymes such as phospholipases, to lyse cell membrane of mucosa cells to obtain a stable homogenate of the mucosa comprising heparin and mucosa proteins and peptides;
(iva) separating heparin and the mucosa proteins and peptides contained in the stable homogenate obtained in step (iiia) by one or more of physical and/or chemical means selected from the group consisting of centrifugation and filtration (microfiltration and/or ultrafiltration), optionally using detergents, and combinations thereof, to obtain a pellet fraction comprising heparin (P) and proteins; and a supernatant fraction (SN) comprising mucosa proteins and peptides;
(v.a') submitting the pellet fraction (P) of (iva) comprising heparin to an alkaline proteolytic process by means of proteolytic enzymes to obtain a mixture of heparin and a protein hydrolysate, submiting the mixture with heparin to one or more precipitation steps, and optionally to one or more of extractive steps, and to one or more of a chromatography to purify the said heparin from the protein hydrolysate; and
(v.b') submitting the supernatant (SN) of (iva) comprising a mixture of solubilized and/or suspended mucosa proteins and peptides to one or more of successive filtration, ultrafiltration steps, solvent extraction, protein precipitation, enzymatic hydrolysis, gel permeation, ion exchange chromatography, size exclusion chromatography, or affinity chromatography steps, being exchangeable the order or sequence of the steps, in order to separate the proteins and peptides according to any of their solubility, isoelectric point and/or molecular weight.

A further aspect of the invention is a method for the obtention of heparin by fractionation of mammalian intestine mucosa, comprising the steps (i), (ii), (iiia), (iva), and (v.a') as defined herein.

A further aspect of the invention is a method for the obtention of proteins and peptides in native state by fractionation of mammalian intestine mucosa, comprising the steps (i), (ii), (iiia), (iva), and (v.b') as defined herein.

Several compositions including compounds of interest are obtained by the method. In particular compositions of or fractions from the mammalian intestine mucosa comprising purified acidic or basic proteins (i.e., enzymes) that are then useful in the common applications they are known for or for any new ones.

Thus, disclosed herein are compositions comprising an acidic fraction of intestine mammal mucosa acidic proteins and peptides which is obtainable by a method comprising steps (i), (ii), (iiia), and (iva) as defined herein, further comprising:
(v.b.1') submitting the supernatant (SN) of step (v.b') comprising a mixture of solubilized and/or suspended mucosa proteins and peptides to filtration through an up to 100 µm cut-off filter in order to retain small particles and lipid residues which were not pelleted in step (iva), to obtain a filtered supernatant comprising most of the mucosa proteins and peptides; and
(v.b.4') submitting the supernatant of step (v.b.1') to an anionic exchange chromatography column to obtain a bound fraction (SNbf') and an unbound fraction (SNubf'), and eluting the bound fraction and then diafilter and concentrate it by means of a tangential flow system through a filter having a cut-off from 1 to 5 KDa to obtain an acidic fraction of the supernatant comprising acidic proteins and peptides.
or alternatively obtainable by a method comprising steps (i), (ii), (iiia), and (iva) as defined herein, further comprising:
(v.b.1') submitting the supernatant (SN) of step (v.b') comprising a mixture of solubilized and/or suspended mucosa proteins and peptides to filtration through an up to 100 µm cut-off filter in order to retain small particles and lipid residues which were not pelleted in step (iva), to obtain a filtered supernatant comprising most of the mucosa proteins and peptides;
(v.b.4a) submitting the supernatant of step (v.b.1') to a cationic exchange chromatography column to obtain a bound fraction (SNbf) and an unbound fraction (SNubf), and eluting the bound fraction and then diafilter and concentrate it by means of a tangential flow system through a filter having a cut-off from 1 to 5 KDa, to obtain a basic fraction of the supernatant comprising basic proteins and peptides; and
(v.b.5a) submitting the unbound fraction (SNubf) of step (v.b.4a) to an anionic exchange chromatography column to obtain a bound fraction (SNubfbf) and an unbound fraction (SNubub), and eluting the bound fraction and then diafilter and concentrate it by means of a tangential flow system through a filter having a cut-off from 1 to 5 KDa, to obtain an acidic fraction of the supernatant comprising acidic proteins and peptides.

Also disclosed herein is a basic fraction of intestine mammal mucosa basic proteins and peptides obtainable by a method comprising steps (i), (ii), (iiia), (iva), (v.b.1'), and (v.b.4') as defined herein to obtain an acidic fraction of the supernatant comprising acidic proteins and peptides; and further comprising:
(v.b.5') submitting the unbound fraction (SNubf') to a cationic exchange chromatography column to obtain a bound fraction (SNubfbf') and an unbound fraction (SNubub'), and eluting the bound fraction then diafilter and concentrate it by means of a tangential flow system through a filter having a cut-off from 1 to 5 KDa to obtain a basic fraction of the supernatant comprising basic proteins and peptides;
or alternatively obtainable by a method comprising steps (i), (ii), (iiia), (iva), (v.b.1'), and (v.b.4a) as defined herein.

Also disclosed herein is a composition SNubub' comprising intestine mammal mucosa proteins and peptides obtainable by a method comprising steps (i), (ii), (iiia), (iva), (v.b.1'), (v.b.4'), and (v.b.5') as defined herein.

Also disclosed herein is a composition SNubub comprising intestine mammal mucosa proteins and peptides obtainable by a method comprising steps (i), (ii), (iiia), (iva), (v.b.1'), (v.b.4a), and (v.b.5a) as defined herein.

Also disclosed herein is as will be illustrated in the examples, a fraction comprising heparin of pharmaceutical grade. Thus, a heparin complying with all the regulatory aspects according to health authorities.

In the context of this methodology, a mixture of native proteins of the mammalian intestine mucosa is obtained with a high yield, including the ones having high enzymatic activity.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

As used herein, the indefinite articles "a" and "an" are synonymous with "at least one" or "one or more." Unless indicated otherwise, definite articles used herein, such as "the" also include the plural of the noun.

The term "about" or "around" as used herein refers to a range of values ± 10% of a specified value. For example, the expression "about 10" or "around 10" includes ± 10% of 10, i.e. from 9 to 11.

Enzyme activity, when indicated, is defined as the moles of substrate converted per unit time = rate × reaction volume. Enzyme activity is a measure of the quantity of active enzyme present and is thus dependent on conditions, (mainly pH and temperature). The SI unit is the katal, 1 katal = 1 mol s-1, but this is an excessively large unit. A more practical and commonly used value is enzyme unit (U) = 1 µmol min-1. 1 U corresponds to 16.67 nanokatals.

The specific activity of an enzyme is another common unit. This is the activity of an enzyme per milligram of total protein (expressed in µmol min-1 mg-1). Specific activity gives a measurement of enzyme purity in the mixture. It is the micro moles of product formed by an enzyme in a given amount of time (minutes) under given conditions per milligram of total proteins. Specific activity is equal to the rate of reaction multiplied by the volume of reaction divided by the mass of total protein. The SI unit is katal/kg, but a more practical unit is µmol/mg·min. Specific activity is a measure of enzyme processivity (the capability of enzyme to be processed), at a specific (usually saturating) substrate concentration, and is usually constant for a pure enzyme.

The enzymatic activities of the enzymes obtained by the described method can also be expressed in units of activity per kilogram of mucosa (expressed in µmol min-1 kg-1). This parameter gives a measurement of the quantity of enzyme extracted from the initial raw material.

The term "Mammalian intestine mucosa" according to this description is the term used in the terminology of the processing of animal sub-products in slaughterhouses, such as visceral material from animals. It includes all the material obtained after the scraping of intestines, namely the small intestine. From this scraping a clean tube/membrane is obtained, and the scrapped material is the so-called mammalian intestine mucosa, a complex mixture that comprises the intestine epithelium, the lamina propria (mucosa) and the muscularis mucosae (mucosa), the microvilli with the brush border and the lumen. This complex mixture is the one that in present application is, in some examples and embodiments, submitted to homogenization and further processed to obtain the enzymes of interest and heparin.

As previously indicated, a first aspect of the invention is a method for the simultaneous obtention of heparin, proteins, and peptides in native state by fractionation of mammalian intestine mucosa, comprising the following steps:
(i) adding to an aqueous extract of mammalian intestinal mucosa a preservative and antioxidant agent to obtain a preserved mucosa, or preserving the extract at a temperature allowing preserving proteins, peptides and heparin in native state;
(ii) optionally diluting the preserved mucosa with up to 100 volumes of deionized water, to obtain a diluted mucosa;
(iii) submitting diluted mucosa of step (ii) or preserved mucosa of (i) to homogenization at a temperature of less than 40 °C, to lyse cell membrane of mucosa cells to obtain a stable homogenate of the mucosa comprising heparin and mucosa proteins and peptides;
(iv) separating heparin and the mucosa proteins and peptides contained in the stable homogenate obtained in step (iii) by one or more of physical and/or chemical means selected from the group consisting of centrifugation, filtration and/or ultrafiltration, optionally using detergents, and combinations thereof, to obtain a pellet fraction comprising heparin (P) and proteins; and a supernatant fraction (SN) comprising mucosa proteins and peptides;
(v.a) submitting the pellet fraction (P) of (iv) comprising heparin to an alkaline proteolytic process by means of proteolytic enzymes to obtain a mixture of heparin and a protein hydrolysate, submiting the mixture with heparin to one or more precipitation steps, and optionally to one or more of extractive steps, and to one or more of a chromatography to purify the said heparin from the protein hydrolysate; and
(v.b) submitting the supernatant (SN) comprising a mixture of solubilized and/or suspended mucosa proteins and peptides to one or more of successive filtration, ultrafiltration steps, protein precipitation and/or gel permeation and/or to ion exchange chromatography steps, being exchangeable the order or sequence of the steps, in order to separate the proteins and peptides according to any of their solubility, isoelectric point and/or molecular weight.

Steps (v.a) and (v.b) are also referenced in this description as (v.P) and (v.SN), respectively, for referring to the managing or further processing of the (P) and (SN) fractions of (iv), which allow the simultaneous obtention of the heparin, and of the proteins and peptides in native state, of mammalian intestine mucosa.

"Simultaneous" is to be understood not only as processed at the same time with the required and adapted equipment, but also including a differential processing in time or place but providing from the same intestine mucosa the two compositions of interest. In other words, meanwhile heparin is obtained most of the other proteins and peptides of interest are preserved and also purified from another fraction also obtained from the same mucosa. This is genuine from the method of the invention, since until now in the process for the obtaining of heparin with a high yield from mammalian intestine mucosa, the proteolytic step applied only allowed to obtain a mixture of peptides besides the heparin; thus, non-functional or native proteins and peptides.

Present inventors have surprisingly found a combination of steps and pre-treatments that in combination allow obtaining both, heparin with a high yield and purity grade, as well as the native proteins and peptides (including functional enzymes) of the mammalian intestine mucosa.

The fractions obtained thereof contain the heparin or the proteins and peptides in several purification grades depending on the type, number and sequence of the physical and/or chemical means applied in step (iv) and the following (v.a) or (v.b). It will be understood that in any case the contents of each of the fractions are profitable products or compositions resulting from the method of the invention, which method allows the simultaneous obtention of heparin and a myriad of native and functional proteins and peptides from mammalian intestine mucosa of slaughtered animals.

In the paragraphs that follow, particular embodiments of the type and number and sequence of these physical and/or chemical means applied are illustrated. The accompanying examples serve also for the purpose of showing the yield of the method for obtaining fractions with noteworthy amounts of heparin while certain enzymes of interest, in native and functional state, are also obtained with noteworthy yields.

In a particular embodiment of the method of the first aspect, the mammalian intestine mucosa is the pig one. Other mammalian species, not including human, are also useful.

In another particular embodiment of the method, the preservative and antioxidant agent in step (i) is sodium metabisulfite, added up to a concentration of 4% w/w, preferably 2% w/w. Equivalent substances can be chosen as a preservative and antioxidant agents.

In another particular embodiment, optionally in combination with any of the method embodiments above or below, (ii) the preserved mucosa is diluted with up to 50 volumes of deionized water, more in particular is diluted with 1 to 25 volumes of deionized water, even more in particular with 1 to 15 volumes of deionized water, and also more in particular with 2 to 4 volumes of deionized water. In a more particular embodiment, instead of deionized water, a same volume of buffer at a pH comprised between 5 and 9 is used, preferably at 7.

In a more particular embodiment of the method, in step (ii) the mucosa is diluted either with deionized water or a buffer, and more in particular both comprising one or more detergents. In even a more particular embodiment the detergents are non-ionic detergents or zwitterionic detergents. Examples of commercially available detergents are selected from the group consisting of Tween 20^{®}, Tween 80, Triton X-100 or X-114, deoxycholic acid, Brij 35 or 58, BigCHAP or deoxy BigCHAP, MEGA 8, MEGA 9 or MEGA 10, octyl beta-glucoside, and combinations thereof.

Steps (iii), (iv), (v.a), (v.b), (v.b.1), (v.b.4) and (v.b.5) are equivalent to steps (iiia), (iva), (v.a'), (v.b'), (v.b.1'), (v.b.4a) and (v.b.5a) respectively. All embodiments defined herein for steps (iii), (iv), (v.a), (v.b), (v.b.1), (v.b.4) and (v.b.5) also apply to steps (iiia), (iva), (v.a'), (v.b'), (v.b.1'), (v.b.4a) and (v.b.5a) respectively.

In a particular embodiment, step (iii) or step (iiia) is performed at a temperature equal to or lower than about 35 °C, or alternatively equal to or lower than about 30 °C, or alternatively equal to or lower than about 25 °C, or alternatively equal to or lower than about 20 °C, or alternatively equal to or lower than about 15 °C, or alternatively equal to or lower than about 10 °C.

In another particular embodiment, step (iii) or step (iiia) is performed at a temperature from 0 to 35 °C, or alternatively from 0 to 30 °C, or alternatively from 0 to 25 °C, or alternatively from 0 to 20 °C, or alternatively from 0 to 15 °C, or alternatively from 0 to 10 °C. In yet another particular embodiment, step (iiia) is performed at a temperature from 0 °C to 8 °C. In yet another particular embodiment, step (iii) is performed at a temperature from 0 °C to 8 °C.

In another particular embodiment of the method, homogenization step (iii) is carried out by a technique selected from the group consisting of mechanical or physical cell lysis of mammalian intestinal mucosa by mechanical disruption, shear fluid forces, high pressure or cavitation.

In another particular embodiment of the method, homogenization step (iiia) is carried out by a technique selected from the group consisting of mechanical or physical cell lysis of mammalian intestinal mucosa by mechanical disruption, shear fluid forces, high pressure or cavitation. In another particular embodiment, the homogeneization step (iiia) is carried out in the presence of detergents or hydrolytic enzymes such as phospholipases to detach bound proteins to membranes or cell structures.

In a more particular embodiment, said homogenization step (iii) is carried out by mechanical disruption of the mucosa cells by means of a rotor-stator homogenizer at the g-force of at least 180 g, preferably 1700 g, for a minimum of 30 seconds, preferably between 2 and 4 minutes.

In a particular embodiment, homogenization step (iiia) is carried out by mechanical disruption of the mucosa cells by means of a rotor-stator homogenizer. More in particular at a g-force of at least about 150 g, about 180g, about 500 g, about 750 g, about 1000 g, about 1250 g, about 1500 g, about 1700 g, about 1750 g or about 2000 g; more in particular from 1500 to 2000 g. More in particular homogenization is carried out at about 1700 g.

In a particular embodiment, homogenization step (iiia) is carried out for at least about 30, about 40, about 50, about 60, about 70, about 80, about 90, about 100, about 110, about 120, about 130, about 140, about 150, about 160, about 170, about 180, about 190, about 200, about 210, about 220, about 230, or about 240 seconds. More in particular the homogenization is carried out for a period from 120 to 240, from 130 to 230, from 140 to220, from 150 to 210, from 160 to 200, or from 170 to 190 seconds, even more in particular from 120 to 240 seconds.

In a more particular embodiment, the homogenization step (iiia) is carried out by mechanical disruption of the mucosa cells by means of a rotor-stator homogenizer at a g-force of at least 180 g, preferably 1700 g, for a minimum of 30 seconds, preferably between 2 and 4 minutes.

Step (iva) comprises separating heparin and the mucosa proteins and peptides contained in the stable homogenate obtained in step (iiia) by one or more of physical and/or chemical means selected from the group consisting of centrifugation, filtration and/or ultrafiltration, optionally using detergents, and combinations thereof, to obtain a pellet fraction comprising heparin (P) and proteins; and a supernatant fraction (SN) comprising mucosa proteins and peptides.

The separation step (iva) may be performed by methods well-known in the art. The skilled in the art will be able to determine the exact conditions of the separation depending on the proteins of interest.

Also, another particular embodiment of the method of the invention comprises carrying out step (iv) of separation by centrifugation in order to obtain a pellet fraction comprising heparin (P); and a supernatant fraction comprising mucosa proteins and peptides, some of them in solution and others in suspension (SN). As previously indicated in the first aspect, these (P) and (SN) fractions are further processed in respective steps (v.P) or (v.a), and (v.SN) or (v.b), both notations being interchangeable.

In a particular embodiment, the centrifugation in (iva) is carried out at a g-force of at least about 150 g, about 500 g, about 750 g, about 1000 g, about 1250 g, about 1500 g, about 1750 g, about 2000 g, about 2250 g, about 2500 g, about 2750 g, about 3000 g, about 3100 g, about 3200 g, about 3300 g, about 3400g or about 3500 g; in particular from 150 to 3500 g, from 500 to 3400 g, from 750 to 3300 g, from 1000 to 3200 g, from 1250 to 3100 g, from 1500 to 3000 g, from 1750 to 2750 g, from 2000 to 2500 g; more in particular from 3000 to 3500 g.

In a particular embodiment the centrifugation in (iva) lasts for at least about 5, about 10, about 15, about 20, about 25, about 30, or about 35 minutes, in particular from 5 to 35, from 10 to 30, or from 15 to 25 minutes; more in particular it lasts for from 20 to30 minutes.

In a particular embodiment the centrifugation in (iva) is performed at a temperature equal to or lower than about 40 °C, about 37 °C, about 35 °C, about 30 °C, about 25 °C, about 20 °C, about 15 °C, about 10 °C, about 8 °C, about 5 °C, about 2 °C, or about 0 °C, in particular at a temperature from 0 to 40 °C, or from 2 to 37 °C, or from 5 to 35 °C, or from 10 to 30 °C, or from 15 to 25 °C, more in particular the centrifugation in (iva) is performed at a temperature from 0 to 25 °C, even more in particular at a temperature from 0 to 8 °C.

In a most particular embodiment, the centrifugation in (iv) is carried out at a g-force of at least 150 g, preferably at 3000 g during at least 5 minutes, preferably 20 minutes and at a temperature below 40°C, in particular below 25 °C, more preferably below 8 °C, to obtain, as indicated, a solid phase containing heparin (thus, a pellet phase (P)); and a liquid phase containing a mixture of mucosa proteins and peptides, some of them in solution, others in suspension (thus, a supernatant phase (SN)).

In a most particular embodiment, the centrifugation in (iva) is carried out at a g-force of at least 150 g, preferably at 3000 g during at least 5 minutes, preferably 20 minutes and at a temperature below 40°C, in particular below 25 °C, more preferably below 8 °C, to obtain, as indicated, a solid phase containing heparin (thus, a pellet phase (P)); and a liquid phase containing a mixture of mucosa proteins and peptides, some of them in solution, others in suspension (thus, a supernatant phase (SN)).

In a more particular embodiment, the centrifugation in (iva) is carried out at a g-force from 3000 to 3500 g for a period of time from 15 to 35 minutes, particularly from 20 to 30 minutes, and at a temperature from 0 to 40 °C, or from 0 to 37 °C, or from 0 to 25 °C, or from 0 to 8 °C.

In a more particular embodiment, the centrifugation in (iva) is carried out at a g-force of about 3134 g for a period of time about 20 minutes and at a temperature below about 8 °C.

In a more particular embodiment, the centrifugation in (iva) is carried out at a g-force of about 3300 g for a period of time of about 30 minutes and at a temperature below about 37 °C, particularly from 0 to 25 °C.

Also, another particular embodiment of the method of the invention comprises carrying out step (iva) of separation by filtration or ultrafiltration in order to obtain a pellet (retentate) fraction comprising heparin; and a supernatant (permeate) fraction comprising mucosa proteins and peptides, some of them in solution and others in suspension. As previously indicated in the first aspect, these (P) and (SN) fractions are further processed in respective steps (v.P) or (v.a'), and (v.SN) or (v.b'), both notations being interchangeable.

In a particular embodiment, the supernatant (SN) obtained in step (iva) comprises one or more of alkaline phosphatase (also referred herein as to ALP), mucin-2 (also referred herein as to MUC2) and lysozyme (also referred herein as to LYZ), more particularly it comprises alkaline phosphatase, mucin-2 and lysozyme. More in particular the SN further comprises one or more of glucagon like peptide-1 (also referred herein as to GLP-1), thymosin beta-4, vaso intestinal peptide, lysozyme, lactotransferrin, regenerating islet-derived protein 3 (Reg III), regenerating islet-derived protein IV (Reg IV), matrix metalloproteinase-9, 72 kDa type IV collagenase, interstitial collagenase, bactericidal permeability-increasing protein, pulmonary surfactant-associated protein D, antibacterial protein PR-39, angiogenin, alkaline phosphatase, mucin-2, trypsin, aminopeptidases, carboxypeptidases, catalase, triacylglycerol lipase, phospholipases, acid sphingomyelinase-like phosphodiesterase, alpha-amylase, alpha-galactosidase, sucrase-isomaltase, peroxiredoxin-6, superoxide dismutase, apolipoprotein A1, annexin-5, galectin-1, diazepam binding inhibitor, immunoglobulins, gastrotropin, and hemoglobin; and more particularly comprises all of them.

As indicated, in a step (v.P) or (v.a), and with the aim of recovering most of the heparin of the homogenate and with a high degree of purity, the pellet (P) of previous embodiments in which a step of centrifugation is applied, is submitted to an alkaline proteolytic process by means of proteolytic enzymes to obtain heparin and a protein hydrolysate. Next, precipitation steps, optional extractive steps, and optional chromatography are carried out to purify heparin.

This further steps of alkaline proteolytic process and heparin purification are equivalent to the standard ones, known as master file batch for the extraction of heparin, and that gives heparin of pharmaceutical grade and a separate fraction comprising a protein hydrolysate that is usually used as animal food additive (as explained in the background section and in previous paragraphs).

Thus, the method of the invention provides a heparin extracted from mammalian intestinal mucosa and a protein hydrolysate, which are obtainable by a method as defined in the first aspect and its embodiment including the alkaline proteolytic process step.

Parallel to the extraction of heparin, in a step (v.SN) or (v.b) in another particular embodiment of the first aspect, also optionally in combination with any of the embodiments above or below, the supernatant fraction (SN) comprising solubilized and/or suspended a mixture of mucosa proteins and peptides when a centrifugation step in (iv) is carried out for its obtention, it is submitted to one or more of successive filtration, ultrafiltration steps, protein precipitation and/or gel permeation and/or to ion exchange chromatography steps, being exchangeable the order or sequence of the steps, in order to separate the proteins and peptides according to any of their solubility, isoelectric point and molecular weight.

This purification process of the SN (i.e., step (v.SN)) comprises, in some embodiments, a separation of all soluble proteins and peptides from the insoluble or suspended ones. The SN is submitted then to a diafiltration process through cassettes or cartridges having a cut-off between 300 kDa and 0.45 µm, preferably 0.1 µm.

The retentate of this diafiltration step (SNr) contains small particles, lipid residues and all the mucosa proteins and peptides which are insoluble in water, or partly water soluble. The latter are bound to other cell structures such as cell membranes or organelles or other insoluble proteins to form aggregates, and they were not pelleted in a centrifugation within step (iv) of the method and, thus are not filterable (i.e., diafilterable).

On the contrary, the permeate of this diafiltration step (SNp) contains all water-soluble proteins and peptides having a molecular weight less than the cut-off of the filter applied in the diafiltration step described above.

In a further purification step, the fraction SNp is further diafiltered and concentrated through a filter having a cut-off between 1 and 5 kDa with the aim of retaining all the soluble proteins and peptides contained in SNp, obtaining a concentrated fraction of soluble proteins and peptides (SNpr).

In a more particular embodiment, when the SNp is diafiltered to obtain the SNpr, the diafiltration is carried out by means of a tangential flow filtration equipment. In even a more particular embodiment, the diafiltration is carried out by substituting the permeate with at least 4 volumes of deionized water or a suitable buffer, preferably between 5 and 10 volumes.

In a particular embodiment, optionally in combination with any of the embodiments above or below, the SNp (or SNpr) comprising solubilized mucosa proteins and peptides is then submitted to additional separation techniques, based according to the isoelectric point of the proteins and peptides. In other words, SNp (or SNpr) comprising solubilized mucosa proteins and peptides is submitted to other of the also included in step (v.b) one or more of successive filtration, ultrafiltration steps, protein precipitation and/or gel permeation and/or to ion exchange chromatography steps, being exchangeable the order or sequence of the steps allowing the separation of said proteins and peptides by means based according to the isoelectric point of the proteins and peptides.

In a particular embodiment of step (v.b), an SNp obtained as indicated in previous paragraphs and comprising the soluble mucosa proteins and peptides is, preferably after having adjusted the pH to 7.0, loaded downflow on a cationic exchange chromatographic column to obtain a bound fraction (SNpbf) and an unbound fraction (SNpubf); and the bound fraction is further eluted with the upflow direction and then diafiltered and concentrated by means of a tangential flow system through a filter having a cut-off from 1 to 5 KDa, preferably of 3 Kda, to obtain a basic fraction (also referred herein as to BASIC FRACTION or BF) comprising basic proteins and peptides. Alternatively, the cationic exchange chromatography can be done in batch, adding from 5 to 15% (w/w) of strong cationic exchange resin to the fraction, preferably 10%.

With this step of cationic exchange chromatography, in particular a strong cationic exchange chromatography, all basic proteins in the SNp are consequently concentrated. In a particular embodiment the elution of the bound fraction (SNpbf) and diafiltration is carried out through a phosphate buffer 50 mM at pH between 6.5 and 7.5.

In another particular embodiment of the method, when the SNp is submitted to the cationic exchange chromatographic column, the unbound fraction (SNpubf) is, preferably after having adjusted the pH to 7.0, loaded on an anionic exchange chromatography column, and further eluted with the upflow direction and then diafiltered and concentrated by means of a tangential flow system through a filter having a cut-off from 1 to 5 Kda, preferably 3 Kda, to obtain an acidic fraction (also referred herein as to ACIDIC FRACTION or AF) comprising acidic proteins and peptides.

With this further step of anionic exchange chromatography, in particular a strong anionic exchange chromatography, all acidic proteins in the SNp are consequently concentrated. In a particular embodiment the elution of the bound fraction and diafiltration is carried out through a phosphate buffer 50 mM at pH between 6.5 and 7.5.

Alternatively, acidic proteins and peptides can be captured before the basic ones.

In a more particular embodiment, the acidic proteins and peptides are captured before the basic ones.

When a step of ion exchange chromatography is employed the rationale behind the separation is the isoelectric point of the proteins or the peptides that, at a predetermined pH gives a protein positively or negatively charged.

Once any of the acidic and basic fractions are obtained, these fractions are in another particular embodiment submitted to additional fractionation steps by means of diafiltration, salting out methods, gel permeation chromatography, cationic and anionic exchange chromatography and combinations thereof.

In another particular embodiment any of the basic or acidic fractions are further submitted to fractionation by means of diafiltration, salting out methods, protein precipitation, solvent extraction, cationic and anionic exchange chromatography, size exclusion chromatography, affinity chromatography and combinations thereof.

Alternatively, all these fractionation steps can be carried out in different order.

With this subsequent fractionation, purified fractions are obtained, even fractions with only one of the native proteins or peptides from the mammalian intestine mucosa in a buffered water solution.

In a particular embodiment of the method, both the basic and acidic fractions are fractionated by molecular weight through a series of tangential flow diafiltrations and concentrations of the related permeates. The fractions will be characterized, in even a more particular embodiment, by the following molecular weight ranges:
More than 300 Kda
Between 100 and 300 Kda
Between 50 and 100 Kda
Between 30 and 50 Kda
Between 10 and 30 Kda
Between 5 and 10 Kda
Between 1 and 5 Kda

In a more particular embodiment, when the method of the invention comprises the further fractionation of the obtained basic and acidic fractions by molecular weight as indicated in the previous embodiment, both the basic and acidic fractions from the step are further fractionated by molecular weight through a gel permeation chromatography.

In an alternative particular embodiment of the method, both the basic and acidic fractions are fractionated by salting out methods, such as more in particular through the addition of ammonium sulphate or by the addition of an organic solvent such as acetone or butanol.

A "salting out method" is to be understood as a step of precipitation of proteins due to the presence in the media of salts that lower the solubility of particular proteins.

In also another alternative particular embodiment of the method, both the basic and acidic fractions are loaded on weak cationic and anionic exchange chromatography columns, respectively, and the single (isolated) proteins and peptides, are eluted by means of ionic strength or pH gradient.

In another embodiment of the present invention, the SNr fraction is treated in order to obtain products containing enzymatic activity having chemical, nutraceutical or pharmaceutical interest.

In a particular embodiment, SNr is treated in order to release water-soluble proteins, having or not enzymatic activity, from their bonds to membranes or other cell structures.

In one particular embodiment, SNr is treated with homogenization (with or without detergent), an/or with hydrolytic enzymes to detach bound proteins from membranes or cell structures. In a particular embodiment the hydrolytic enzymes comprise one or more of phospholipases and proteases. All released proteins and peptides are further purified by the same methods used for SNp in the series of one or more of successive filtration, ultrafiltration steps, protein precipitation and/or gel permeation and/or to ion exchange chromatography steps of (v.b) (i.e., (v.SN)).

As will be illustrated in the examples below, with this method of the first aspect in all its variations, particular fractions containing enzymes of interest in active form were obtained. Thus, mixtures of enzymes from the mammal intestine mucosa that perform and function according to their activities.

In a particular embodiment of the method of the first aspect, for the simultaneous obtention of heparin, proteins and peptides in native state by fractionation of mammalian intestine mucosa, is a method comprising the following steps:
(i) adding to an aqueous extract of mammalian intestinal mucosa a preservative and an antioxidant agent to obtain a preserved mucosa, or preserving the extract at a temperature allowing preserving proteins, peptides and heparin in native state;
(ii) optionally diluting the preserved mucosa with up to 100 volumes of deionized water, to obtain a diluted mucosa;
(iii) submitting diluted mucosa of step (ii) or preserved mucosa of (i) to homogenization at a temperature of less than 40 °C, to lyse cell membrane of mucosa cells to obtain a stable homogenate of the mucosa comprising heparin and mucosa proteins and peptides;
(iv) separating heparin and the mucosa proteins and peptides contained in the stable homogenate obtained in step (iii) by one or more physical and/or chemical means selected from the group consisting of centrifugation, filtration and/or ultrafiltration, optionally using detergents, and combinations thereof, to obtain a pellet phase (P) comprising heparin; and a supernatant (SN) fraction comprising mucosa proteins and peptides;
(v.a) submitting the pellet fraction (P) of (iv) comprising heparin to an alkaline proteolytic process by means of proteolytic enzymes to obtain a mixture of heparin and a protein hydrolysate, submiting the mixture with heparin to one or more precipitation steps, and optionally to one or more of extractive steps, and to one or more of a chromatography to purify the said heparin from the protein hydrolysate; and
(v.b.1) submitting the supernatant (SN) comprising a mixture of solubilized and/or suspended mucosa proteins and peptides to filtration through an up to 100 µm cut-off filter in order to retain small particles and lipid residues which were not pelleted in step (iv), to obtain a filtered supernatant comprising most of the mucosa proteins and peptides;
(v.b.2) diafiltering the filtered supernatant through a filter having a cut-off of less than 0.8 µm to obtain a permeate (SNp) comprising the mucosa soluble in water proteins and peptides, and a retentate (SNr) with non-soluble in water proteins and peptides (i.e., those that were suspended in the crude supernatant SN);
(v.b.3) optionally further diafiltering the permeate (SNp) to concentrate the soluble proteins and peptides (SNpr);
(v.b.4) submitting SNp (or SNpr) to a cationic exchange chromatography column to obtain a bound fraction (SNpbf) and an unbound fraction (SNpubf), and eluting the bound fraction and then diafilter and concentrate it by means of a tangential flow system through a filter having a cut-off from 1 to 5 KDa, to obtain a basic fraction of the filtered supernatant comprising basic proteins and peptides; and
(v.b.5) submitting the unbound fraction (SNpubf) to an anionic exchange chromatograpy column, and further eluting it, and diafiltering and concentrating it by means of a tangential flow system through a filter having a cut-off from 1 to 5 KDa, to obtain an acidic fraction of the filtered supernatant comprising acidic proteins and peptides;
being exchangeable the order or sequence of the cationic and anionic exchange chromatography.

In a specific embodiment, step (iv) of separating heparin fraction (P) from the supernatant fraction (SN) is carried out by a centrifugation of the stable homogenate obtained in step (iii).

In another more particular embodiment, optionally in combination with any of the embodiments above or below, step (v.SN) or (v.b) further comprises submitting any of the basic or acidic fractions obtained according to steps (v.b.4) and (v.b.5) to further fractionation steps, such as fractionation by molecular weight, by means of additional diafiltration, salting out methods, additional cationic and anionic exchange chromatography, and combinations thereof. This later embodiment aims to achieve the high purification of each of the peptides and proteins in the fractions, if required.

In even a more particular embodiment of the method including in step (v.b) the further fractionation by molecular weight of any of the basic or acidic fractions of (v.b.4) and (v.b.5), the peptides and proteins are fractionated in a step (v.b.6) according to molecular weights ranges selected from more than 300 KDa, between 100 and 300 KDa, between 50 and 100 KDa, between 30 and 50 KDa, between 10 and 30 KDa, between 5 and 10 KDa, and between 1 and 5 KDa.

With the tangential flow the samples were, thus first concentrated filtering out the low molecular weight compounds (i.e., water and other compounds), and then by means of the further selected fractionations, particular peptides and proteins within a known molecular weight can be recovered.

In one particular embodiment, optionally in combination with any of the embodiments above or below, the retentate (SNr) of (v.b.2) is further treated in a sub-step (v.b.7) with an homogenization step (with or without detergent), and/or with hydrolytic enzymes, such as one or more of phospholipases and proteases to detach the bound proteins of interest from the membranes or other cell structures. All released proteins and peptides are further purified by the same methods used for SNp and disclosed in steps (v.b.3) to (v.b.6). The step of homogenization of the retentate (SNr) is, in a particular embodiment, carried out in the same way that homogenization of step (iii) in the method is carried out. Thus, it is carried out with a technique selected from the group consisting of mechanical or physical cell lysis of mammalian intestinal mucosa by mechanical disruption, shear fluid forces, high pressure or cavitation.

In another embodiment, the method of the invention further comprises:
(v.b.4') submitting the supernatant of step (v.b.1') to an anionic exchange chromatography column to obtain a bound fraction (SNbf') and an unbound fraction (SNubf'), and eluting the bound fraction and then diafilter and concentrate it by means of a tangential flow system through a filter having a cut-off from 1 to 5 KDa to obtain an acidic fraction of the supernatant comprising acidic proteins and peptides.

In another embodiment, the method of the invention further comprises:
(v.b.5') submitting the unbound fraction (SNubf') of step (v.b.4') to a cationic exchange chromatography column to obtain a bound fraction (SNubfbf') and an unbound fraction (SNubub'), and eluting the bound fraction then diafilter and concentrate it by means of a tangential flow system through a filter having a cut-off from 1 to 5 KDa to obtain a basic fraction of the supernatant comprising basic proteins and peptides.

In another embodiment, the method of the invention further comprises:
(v.b.4a) submitting the supernatant of step (v.b.1') to a cationic exchange chromatography column to obtain a bound fraction (SNbf) and an unbound fraction (SNubf), and eluting the bound fraction and then diafilter and concentrate it by means of a tangential flow system through a filter having a cut-off from 1 to 5 KDa, to obtain a basic fraction of the supernatant comprising basic proteins and peptides.

In another embodiment, the method of the invention further comprises:
(v.b.5a) submitting the unbound fraction (SNubf) of step (v.b.4a) to an anionic exchange chromatography column to obtain a bound fraction (SNubfbf) and an unbound fraction (SNubub), and eluting the bound fraction and then diafilter and concentrate it by means of a tangential flow system through a filter having a cut-off from 1 to 5 KDa, to obtain an acidic fraction of the supernatant comprising acidic proteins and peptides.

In a more particular embodiment, the anionic exchange chromatography is performed before the cationic exchange chromatography.

In another embodiment, the method of the invention further comprises:
(v.b.2) diafiltering the supernatant of step (v.b.1') through a filter having a cut-off of less than 0.8 µm to obtain a permeate (SNp) comprising the mucosa soluble in water proteins and peptides, and a retentate (SNr) with non-soluble in water proteins and peptides; and
(v.b.3) optionally diafiltering the permeate (SNp) to concentrate the soluble proteins and peptides (SNpr).

In those embodiments comprising step (v.b.2), the next step (v.b.4') or (v.b.4a) is performed with the supernatant SNp or SNr of step (v.b.2)., particularly with the supernatant SNp. In those embodiments comprising steps (v.b.2) and (v.b.3), the next step (v.b.4') or (v.b.4a) is performed with the supernatant SNp or SNpr of step (v.b.3), particularly with the supernatant SNp.

In a particular embodiment, the method for the obtention proteins and peptides in native state by fractionation of mammalian intestine mucosa comprises the steps (i), (ii), (iiia), (iva), (v.b.1'), and (v.b.4') as defined herein. More particularly, it comprises the steps (i), (ii), (iiia), (iva), (v.b.1'), (v.b.2), (v.b.3), and (v.b.4') as defined herein.

In a particular embodiment the method for the obtention proteins and peptides in native state by fractionation of mammalian intestine mucosa comprises the steps (i), (ii), (iiia), (iva), (v.b.1'), (v.b.4a) and (v.b.5a) as defined herein. More particularly, it comprises the steps (i), (ii), (iiia), (iva), (v.b.1'), (v.b.2), (v.b.3), (v.b.4a) and (v.b.5a) as defined herein.

In a particular embodiment the method for the obtention proteins and peptides in native state by fractionation of mammalian intestine mucosa comprises the steps (i), (ii), (iiia), (iva), (v.b.1'), (v.b.4') and (v.b.5') as defined herein. More particularly, it comprises the steps (i), (ii), (iiia), (iva), (v.b.1'), (v.b.2), (v.b.3), (v.b.4') and (v.b.5') as defined herein.

In a particular embodiment the method for the obtention proteins and peptides in native state by fractionation of mammalian intestine mucosa comprises the steps (i), (ii), (iiia), (iva), (v.b.1'), and (v.b.4a) as defined herein. More particularly, it comprises the steps (i), (ii), (iiia), (iva), (v.b.1'), (v.b.2), (v.b.3) and (v.b.4a) as defined herein.

In a particular embodiment the method for the obtention proteins and peptides in native state by fractionation of mammalian intestine mucosa comprises the steps (i), (ii), (iiia), (iva), (v.b.1'), (v.b.4a), (v.b.5a) and (v.b.a) as defined herein. More particularly, it comprises the steps (i), (ii), (iiia), (iva), (v.b.1'), (v.b.2), (v.b.3) (v.b.4a), (v.b.5a) and (v.b.a) as defined herein.

In a particular embodiment the method for the obtention proteins and peptides in native state by fractionation of mammalian intestine mucosa comprises the steps (i), (ii), (iiia), (iva), (v.b.1'), (v.b.4'), (v.b.5') and (v.b.a) as defined herein. More particularly, it comprises the steps (i), (ii), (iiia), (iva), (v.b.1'), (v.b.2), (v.b.3) (v.b.4'), (v.b.5') and (v.b.a) as defined herein.

As indicated, also disclosed herein are compositions (i.e., fractions/extracts of intestine mammal mucosa) comprising intestine mammal mucosa proteins and peptides obtainable by a method as defined in the first aspect or in any of its embodiments.

Thus, this can be disclosed as a composition (i.e., a fraction/extract of intestine mammal mucosa) comprising intestine mammal mucosa proteins and peptides obtainable by a method comprising:
(i) adding to an aqueous extract of mammalian intestinal mucosa a preservative and an antioxidant agent to obtain a preserved mucosa, or preserving the extract at a temperature allowing preserving proteins, peptides and heparin in native state;
(ii) optionally diluting the preserved mucosa with up to 100 volumes of deionized water, to obtain a diluted mucosa;
(iii) submitting diluted mucosa of step (ii) or preserved mucosa of (i) to homogenization at a temperature of less than 40 °C, to lyse cell membrane of mucosa cells to obtain a stable homogenate of the mucosa comprising heparin and mucosa proteins and peptides;
(iv) separating heparin and the mucosa proteins and peptides contained in the stable homogenate obtained in step (iii) by one or more physical and/or chemical means selected from the group consisting of centrifugation, filtration and/or ultrafiltration, optionally using detergents, and combinations thereof, to obtain a pellet phase (P) comprising heparin; and a supernatant (SN) fraction comprising mucosa proteins and peptides;
(v.a) submitting the pellet fraction (P) of (iv) comprising heparin to an alkaline proteolytic process by means of proteolytic enzymes to obtain a mixture of heparin and a protein hydrolysate, submiting the mixture with heparin to one or more precipitation steps, and optionally to one or more of extractive steps, and to one or more of a chromatography to purify the said heparin from the protein hydrolysate; and
(v.b.1) submitting the supernatant (SN) comprising a mixture of solubilized and/or suspended mucosa proteins and peptides to filtration through an up to 100 µm cut-off filter in order to retain small particles and lipid residues which were not pelleted in step (iv), to obtain a filtered supernatant comprising most of the mucosa proteins and peptides;
(v.b.2) diafiltering the filtered supernatant through a filter having a cut-off of less than 0.8 µm to obtain a permeate (SNp) comprising the mucosa soluble in water proteins and peptides, and a retentate (SNr) with non-soluble in water proteins and peptides (i.e., those that were suspended in the crude supernatant SN);
(v.b.3) optionally further diafiltering the permeate (SNp) to concentrate the soluble proteins and peptides (SNpr);
(v.b.4) submitting SNp (or SNpr) to a cationic exchange chromatography column to obtain a bound fraction (SNpbf) and an unbound fraction (SNpubf), and eluting the bound fraction and then diafilter and concentrate it by means of a tangential flow system through a filter having a cut-off from 1 to 5 KDa, to obtain a basic fraction of the filtered supernatant comprising basic proteins and peptides; and
(v.b.5) submitting the unbound fraction (SNpubf) to an anionic exchange chromatograpy column, and further eluting it, and diafiltering and concentrating it by means of a tangential flow system through a filter having a cut-off from 1 to 5 KDa, to obtain an acidic fraction of the filtered supernatant comprising acidic proteins and peptides;
being exchangeable the order or sequence of the cationic and anionic exchange chromatography.

In a particular embodiment of said composition comprising intestine mammal mucosa proteins and peptides, the intestine mammal mucosa proteins and peptides are selected from the group consisting of brush border enzymes, antimicrobial peptides, antioxidant enzymes, hormone peptides, proteins participating in defence and/or healing processes in the intestine mucosa, and combinations thereof, as revealed by literature, transcriptomic, and proteomic analysis of the pig intestine mucosa.

These compositions are thus isolated fractions or extracts of the mammal intestine mucosa comprising the compounds of interest.

In a more particular embodiment of the compositions of the second aspect, they comprise brush border enzymes selected from glycosidases, peptidases, phosphatases, lipases, and combinations thereof.

Brush border of the mammalian intestine, in particular of the small intestine, is the microvilli-covered surface of the epithelium where absorption takes place with microvilli of approximately 100 nm in diameter and varying from approximately 100 to 2000 nm in length. In particular, the brush borders of the intestinal lining are the site of terminal carbohydrate and protein digestion. The microvilli that constitute the brush border have enzymes for this final part of digestion anchored into their apical plasma membrane as integral membrane proteins. These enzymes are found near to the transporters that will then allow absorption of the digested nutrients.

In even a more particular embodiment, the composition comprises phosphatases, more in particular selected from phytases, alkaline phosphatase and mixtures thereof.

In another particular embodiment, optionally in combination with the embodiments above or below of the compositions they comprise glycosidases. Examples of particular glycosidase are selected from the group consisting of maltase, maltase-glucoamylase, sucrase-isomaltase, alpha-galactosidase, lactase, dextrinase, trehalase, lysozyme and combinations thereof.

In another particular embodiment, optionally in combination with the embodiments above or below of the compositions of the invention, they comprise peptidases selected from the groups consisting of carboxypeptidases, aminopeptidases, endopeptidases, enteropeptidase and dipeptidase.

In another particular embodiment, optionally in combination with the embodiments above or below of the compositions of the invention, they comprise lipases selected from the groups consisting of triacylglycerol lipase, phospholipases, ceramidases and sphingomyelinases.

In another particular embodiment, the disclosure relates to a composition with mucosa proteins and peptides, which comprises:
- one or more of the enzymes selected from Alkaline Phosphatase, Phytase, Alpha-galactosidase, Lysozyme, Aminopeptidase, Carboxipeptidase, Sucrase-Isomaltase, Maltase-Glucoamylase, Alpha-Amylase, Diaminoxidase, Triacylglycerol lipase and Phospholipase; and/or
- antioxidant enzymes selected from Catalase, Glutathione peroxidase, Superoxide dismutase, and combinations thereof; and/or
- mucosa proteins participating in mucosa defence and healing selected from Mucins, Actin, Secretory Immunoglobulin A, Trefoil Factor Family proteins, Thymosin beta 4, Lactoferrin; Apolipoprotein A-I, Annexin A5, Annexin A1, Galectin-1, and combinations thereof; and/or
- peptides with antimicrobial activity selected from Bactericidal permeability increasing protein, Regenerating islet-derived 4, Defensin, Antibacterial peptide PMAP-23, Pulmonary surfactant-associated protein D; Regenerating islet-derived 3, Diazepam-binding inhibitor, Antibacterial peptide 3910, Antibacterial peptide NK-lysin; Liver-expressed antimicrobial peptide 2, Gastric inhibitory polypeptide (7-42), Reactive oxygen species modulator 1, and combinations thereof; and/or
- peptide with hormone activity selected from peptide YY, GLP-1, GLP-2, Gastrin, Cholecystokinin, Gastric inhibitory polypeptide, Vasoactive intestinal peptide, Neuropeptide Y, Ghrelin; Secretin; Galanin, and combinations thereof.

As mentioned above, the disclosure also relates to a composition comprising an acidic fraction of intestine mammal mucosa acidic proteins and peptides which is obtainable by a method comprising steps (i), (ii), (iiia), (iva), (v.b.1'), and (v.b.4') as defined herein.

As mentioned above, the disclosure also relates to a composition comprising an acidic fraction of intestine mammal mucosa acidic proteins and peptides which is obtainable by a method comprising steps (i), (ii), (iiia), (iva), (v.b.1'), (v.b.4a) and (v.b.5a) as defined herein.

In particular, the composition comprising an acidic fraction of intestine mammal mucosa acidic proteins and peptides comprises one or more of Pro-glucagon, Glucagon like peptide-1 (GLP-1), Thymosin beta-4 and Vasointestinal peptide. More in particular, the AF comprises Pro-glucagon, Glucagon like peptide-1 (GLP-1), Thymosin beta-4 and Vasointestinal peptide.

The disclosure also relates to a composition comprising a basic fraction of intestine mammal mucosa basic proteins and peptides which is obtainable by a method comprising steps (i), (ii), (iiia), (iva), (v.b.1'), (v.b.4'), and (v.b.5') as defined herein.

The disclosure also relates to a composition comprising a basic fraction of intestine mammal mucosa basic proteins and peptides which is obtainable by a method comprising steps (i), (ii), (iiia), (iva), (v.b.1'), and (v.b.4a), as defined herein.

In particular, the composition comprising a basic fraction of intestine mammal mucosa basic proteins and peptides comprises lysozyme. More in particular, it comprises one or more of lysozyme, Lactotrasferrin, 72 kDa type IV collagenase, Interstitial collagenase, Matrix metalloproteinase-9, Regenerating family member III / regenerating islet-derived protein 3 (Reg III), Regenerating family member IV / regenerating islet-derived protein IV (Reg IV), Antibacterial protein PR-39, Angiogenin, Phosphoinositide phospholipase C, Phospholipase D, and Pulmonary surfactant-associated protein D, even more particularly it comprises all of them.

In a particular embodiment, the composition comprising a basic fraction of intestine mammal mucosa basic proteins and peptides is subfractioned by washing with buffers of different concentrations. In a more particular embodiment, the washing is with buffers of different concentrations of NaCl. More in particular, a subfraction BF2 is obtained by washing the resin with a buffer of about 2% NaCl. In particular, BF2 comprises lysozyme, in particular with an activity of 150 U-FIP/mL. More in particular, BF2 comprises one or more of lysozyme, lactotransferrin, phosphoinositide phospholipase C, phospholipase D, antibacterial protein PR-39, regenerating family member 4 and angiogenin. In another particular embodiment, a subfraction BF7 is obtained by further washing the resin with a buffer of about 7% NaCl. In a particular embodiment, BF7 comprises one or more of 72 kDa type IV collagenase, Interstitial collagenase, Matrix metalloproteinase-9, Regenerating family member III / regenerating islet-derived protein 3 (Reg III) and Pulmonary surfactant-associated protein D, and even more particularly comprises all of them.

The disclosure also relates to a composition SNubub' comprising intestine mammal mucosa proteins and peptides which is obtainable by a method comprising steps (i), (ii), (iiia), (iva), (v.b.1'), (v.b.2), (v.b.3), (v.b.4'), and (v.b.5') as defined herein.

In particular, the SNubub' comprises alkaline phosphatase (ALP) and mucin-2 (MUC2). More in particular, the SNubub' comprises one or more of ALP, MUC2, aminopeptidases, carboxypeptidases, catalase, triacylglycerol lipase, phospholipases, acid sphingomyelinase-like phosphodiesterase, alpha-amylase, alpha-galactosidase, sucrase-isomaltase, peroxiredoxin-6, superoxide dismutase, Apolipoprotein A1, Annexin-5, Galectin-1, Diazepam binding inhibitor, Immunoglobulin M, secretory Immunoglobulin A, Gastrotropin, Thymosin beta-4 and Hemoglobin, and more particularly comprises all of them.

In more particular embodiments, SNubub' is further fractionated by means of successive filtration, and ultrafiltration steps, solvent extraction, protein precipitation, and/or gel permeation and affinity chromatography steps.

The disclosure also relates to a composition SNubub comprising intestine mammal mucosa proteins and peptides obtainable by a method comprising steps (i), (ii), (iiia), (iva), (v.b.1'), (v.b.4a), and (v.b.5a) as defined herein.

The disclosure also relates to a composition SNububaq comprising intestine mammal mucosa proteins and peptides obtainable by a method comprising steps (i), (ii), (iiia), (iva), (v.b.1'), (v.b.4'), and (v.b.5') as defined herein to obtain a SNubub' fraction; and further comprising:
(v.b.a) submitting the unbound fraction (SNubub') to an extraction treatment with an organic solvent, obtaining an aqueous phase (SNububaq).

More particularly the invention also relates to a composition SNububaq comprising intestine mammal mucosa proteins and peptides obtainable by a method comprising steps (i), (ii), (iiia), (iva), (v.b.1'), (v.b.2), (v.b.3), (v.b.4'), (v.b.5') and (v.b.a) as defined herein.

In a particular embodiment, SNubub' is submitted to an extraction treatment with an organic solvent (setp v.b.a). More in particular the organic solvent is selected from the group consisting of heptanol, pentanol, 4-tert-Butylcatechol, limonene, cyclohexane, and mixtures thereof, more in particular it is butanol from 20 to 60% (w/w), even more in particular it is butanol at about 40% (w/w). More in particular, the extraction treatment lasts for about 5 minutes and is performed at about 37°C.

In a particular embodiment, the aqueous phase of the obtained extract (SNububaq) comprises alkaline phosphatase and mucin-2. In a more particular embodiment, Snububaq comprises alkaline phosphatase with an activity of about 10.7 DEA U/mL. In a more particular embodiment, Snububaq comprises one or more of Alkaline phosphatase, Mucin-2, Immunoglobulin M, Immunoglobulin A, Sucrase-lsomaltase, Alpha-amylase, Hemoglobin, Aminopeptidases, Maltase-glucoamylase, Catalase, Carboxypeptidases, Alpha-galactosidase, Peroxiredoxin-6, Superoxide dismutase, Acid sphingomyelinase-like phosphodiesterase, Diazepam binding inhibitor, Gastrotropin, Apolipoprotein-1, Annexin-5 and Galectin-1, and more particularly comprises all of them.

In another particular embodiment, the Snububaq is submitted to a treatment with a chemical agent for precipitation such as acetone, ammonium sulphate, PEG, Aluminium, and butanone to obtain a precipitate and a supernatant (Snububaqsn). More in particular the organic solvent is acetone, more in particular it is acetone from 20 to 40% (w/w), more in particular it is acetone around 30% (w/w).

In another particular embodiment, the resulting supernatant (Snububaqsn) is further submitted to a treatment with a chemical agent for precipitation such as acetone, ammonium sulphate, PEG, Aluminium, and butanone to obtain a precipitate (Snububaqsnp or ALPp) and a supernatant (Snububaqsnsn or MUC2sn). More in particular the organic solvent is acetone, more in particular it is acetone from 50 to 99% (w/w), more in particular it is acetone from 60% to 90% (w/w), more in particular it is acetone 80%. In a particular embodiment, the acetone treatment is done at a temperature between 25 and 45 °C, in a more particular embodiment between 35 and 40 °C, in a more particular embodiment at 37 °C. In a more particular embodiment, the resulting precipitate (ALPp) comprises ALP. In another particular embodiment, the resulting supernatant (MUCsn) comprises MUC2.

In another embodiment, the ALPp is further processed by means of affinity chromatography to obtain purified ALP. In particular the purified ALP is a pharmaceutical grade protein suitable for medical applications. More in particular, the purified ALP is obtained with a yield of around 38840 DEA Units of alkaline phosphatase activity per kg of mucosa (one DEA unit will hydrolyse 1 µmol of p-nitrophenyl phosphate per minute at pH 9.8 at 37°C, as known by the skilled person).

In another embodiment, the MUC2sn is further purified by means of affinity chromatography to obtain pharmaceutical grade proteins suitable for medical applications. In particular the purified MUC2 is a pharmaceutical grade protein suitable for medical applications. More in particular, the purified MUC2 is obtained with a yield of around 0.16 g of N-acetylneuraminic acid per kg of mucosa corresponding to sialylated glycoproteins like mucin-2.

### Examples

### Example 1a. Transcriptomic analysis of porcine intestinal mucosa

A transcriptomic analysis was performed to explore the complete set of transcripts of pig intestinal mucosa and characterize the corresponding proteome.

### Materials and methods:

### RNA extraction

9 samples of porcine intestinal mucosa were collected at the slaughterhouse and stored at -80 °C until analysis. Total RNA was extracted using RNeasy Plus Mini Kit (ref: 74134). RNA concentration was quantified by fluorescence, and quality was verified by electrophoresis.

### Libraries preparation and sequencing

Transcripts were purified by poly(A)-tail selection. 9 mRNA libraries were prepared using Illumina TruSeq stranded mRNA kit. Libraries were amplified by PCR, and concentration and quality were verified. Libraries were sequenced using Illumina Novaseq 6000 on one lane of SP flow-cell, 2x100bp (Kit version NV2864788-RGSBS).

### Bioinformatic analysis

Gene expression was studied using the reference genome of Sus scrofa11.1 on iGenome (https://emea.support.illumina.com/sequencing/sequencing_software / igenome.html). Reads were mapped against the reference genome using Bowtie v2.0.5 (http://bowtie-bio.sourceforge.net/bowtie2/index.shtml) and TopHat v2.0.6 (http://tophat.cbcb.umd.edu/). Cufflink v2.1.1 (http://cufflinks.cbcb.umd.edu/) was used to estimate abundance of aligned reads, and test for differential expression and regulation transcriptome-wide. The package Cummerbund v2.7.2 (http://compbio.mit.edu/cummeRbund/) was used to produce graphics representations of the differential analysis.

### Results:

A total of 120,814,076 transcripts were read, and 104,992,005 of them were mapped against the reference genome. From the mapped reads, more than 16,000 genes were annotated and their expression was estimated.

### Example 2a. Proteomic analysis of porcine intestinal mucosa samples of example 2b.

A proteomic analysis was performed to map the proteome of the porcine intestinal mucosa and complement the transcriptomic data.

### Materials and methods:

### Protein extraction

Samples of porcine intestinal mucosa were collected at the slaughterhouse and stored at - 80°C until analysis. A representative sample was weight of at -20 °C (106.4mg) in an Eppendorf tube and TES buffer (Tris-HCl 10 mM, EDTA 1 mM, sucrose 0.25mM, pH 6.9) was added to give a concentration of 100mg/mL. The sample was kept on ice and homogenized 2x10sec in a tissue homogenisator (TissueLyser LT, Qiagen) using metal beads, keeping the sample on ice in-between homogenization. The homogenized sample was centrifuges at 4 °C, 15.000 x *g* for 15 min (Eppendorf Centrifuge) and the supernatant was collected. The protein concentration was determined using the BCA method with BSA as standard (Pierce BSA Protein Assay Kit). The protein concentration was determined to 5 µg/µl.

### Protein digestion and purification

In all, 4 samples of 10 µg protein each, where reduced with DTT, alkylated with iodoaceamide and digested with trypsin according to the "In-solution trypsin digestion and guanidination kit" (ThermoScientific). Two of the samples where guanidinated using O-methylisourea hemisulfate. Then, peptides were purified for all 4 samples using "Peptide Desalting Spin Columns" (Pierce, see manufacturers manual). After desalting, the samples were evaporated to dryness and dissolved in HPLC buffer A (2% acetonitrile, 0.1% formic acid in MilliQ water) prior to LC-MS analysis.

### Mass spectrometry analysis

Peptide samples were analyzed on a Bruker TimsTOFPro equipped with a Bruker NanoElute LC-system. Peptides were separated using a 90 min reverse phase gradient, with solvent A: 2% acetonitrile, 0.1% formic acid in MilliQ water, and solvent B: acetonitrile, 0.1% formic acid. The reverse phase column was a BrukerFIFTEEN (L=15cm, ID=75µm, C18, 1.9µm, 120Å). The mass spectrometer was run in DDA PASEF mode.

### Database searching:

Datafiles were merged and searched in PEAKS XPro using the UniProt procine proteomics database (Proteomics ID UP000008227, downloaded 2021.03.05) with the Mammalian SwissProt database as contamination database. Error tolerance: 20ppm precursor mass, 0.05Da fragment ion. Fixed PTM: carmidomethylation. Variable PTMs; oxidation (M), acetylation (N-tern), guanidination. False discovery rate (FDR) was set to 0.5%.

### Results:

Proteomics analysis of porcine intestinal mucosa revealed 2810 identified protein groups, with false discovery rate (FDR) of 0.5%. The data from this experiment was crossed with the data obtained from the transcriptomic analysis to conform a comprehensive proteome of the pig intestinal mucosa. From this, a list of candidate proteins was selected based on their abundance in the mucosa, their molecular characteristics, and their potential commercial application.

### Example 2b. Industrial process (I) for obtaining heparin and by-side products (i.e., native proteins and peptides) from mammalian intestine mucosa.

A method for the simultaneous isolation of heparin and selected native proteins and peptides, from mammalian intestinal mucosa, was performed as follows.

The method generically included the following steps:
(i) An aqueous extract of a mammalian intestinal mucosa was obtained (i.e., separating the mucosa of the intestine with standard methods and dissolving/suspending it in water or in an aqueous buffer); and then to said aqueous extract of mammalian intestinal mucosa (hereinafter called MUCOSA) sodium metabisulfite was added up to a concentration of 2%. Equivalent substances can be chosen as a preservative and antioxidant agent;
(ii) MUCOSA was further diluted with 2 volumes of deionized water. The output of this procedure yielded an intermediate hereinafter called DILUTED MUCOSA. Alternatively, the diluted mucosa could have been obtained by diluting the MUCOSA with an aqueous solution, comprising one of the following detergents Tween 20, Tween 80, Triton X-100 or X-114, deoxycholic acid, Brij 35 or 58, BigCHAP or deoxy BigCHAP, MEGA 8, MEGA 9 or MEGA 10, Octyl beta-glucoside;
(iii) DILUTED MUCOSA was submitted to homogenization by a rotor-stator homogenizer at the G-force of 1700 g, during 2 to 4 minutes at a temperature between 2°C and 8°C to lyse the cell membranes and to obtain a stable homogenate of the mucosa comprising heparin and mucosa proteins and peptides (herein after called HOMOGENATE). Alternative homogenization modes can be performed by other techniques selected from the group consisting of mechanical or physical cell lysis of mammalian intestinal mucosa by mechanical disruption, shear fluid forces, high pressure or cavitation. Another alternative homogenization technique relates to the addition of detergents;
(iv) Heparin and the mucosa proteins and peptides contained in the HOMOGENATE obtained in step (iii) were separated by centrifugation in order to obtain liquid and solid phases, each comprising different proportion of one or more of heparin, and other mucosa proteins and peptides. The centrifugation was carried out at a G-force of 3134 g for 20 minutes and at a temperature below 8°C to obtain: a solid phase containing heparin, (hereinafter called PELLET or P), and a liquid phase containing a mixture of mucosa proteins and peptides (hereinafter called SUPERNATANT or SN).
(v.P) The PELLET was then submitted to the standard extraction method to obtain and purify heparin for pharmaceutical applications (This step can also be labelled as (iv.a), as previously indicated). In brief, the pellet was treated with an enzymatic alkaline proteolysis at 58°C and pH 7.7-8 during 2 hours. After the hydrolysis, pH was decreased to 6.6 and temperature was increased to 90°C and maintained during 15 minutes to precipitate the fat. The product was centrifuged at a G-force of 3134 g for 15 minutes, and the supernatant was decantated and filtrated through a 300 µm filter to separate the fat fraction. Then, an anion exchange resin was added to the supernatant to capture the heparin. The mixture was kept under stirring overnight, and resin was separated from the liquid with a mesh filter, and rinsed with osmotized water. Proteins were eluted from the resin with three successive additions of NaCl 3.5% (w/v) at 45°C for 30 minutes each. Finally, heparin was eluted with three successive additions of NaCl 18%, 20%, and 20% at 45°C for 30 minutes each. The 3 heparin eluates were combined and analyzed yielding an average of 0,047 MIU of heparin activity per Kg of mucosa (one unit of heparin is the quantity of heparin required to keep 1 ml of cat's blood fluid for 24 hours at 0°C; it is equivalent approximately to 0.002 mg of pure heparin, as known by the skilled person).
(v.SN) (or (v.b) as previously indicated) The SUPERNATANT was submitted to successive filtration, ultrafiltration steps, protein precipitation and/or gel permeation and ion exchange chromatography steps in order to separate the proteins and peptides according to their solubility, isoelectric point and molecular weight (see particular steps (iv.SN.1) to (iv.SN.7) as follows).
(v.SN.1) SUPERNATANT was gross filtered through a 100 µm cut-off filter in order to retain small particles and lipid residues which are lighter and, consequently, not pelleted. The intermediate obtained by this procedure was hereinafter called FILTERED SUPERNATANT
(v.SN.2) The FILTERED SUPERNATANT was then diafiltered by means of a tangential flow filtration equipment through a filter having a cut-off of less than 0.1 µm. The diafiltration was carried out by substituting the permeate with at least 4 volumes of a buffer, preferably between 5 and 7 volumes. The aim of this step was to recover both the retentate (SNr) and the permeate (SNp).
(v.SN.3) The permeate (SNp) was further diafiltered by means of a tangential flow filtration equipment through a filter having a cut-off less 3 kDa in order to concentrate it (SNpr).
(v.SN.4) The fraction SNpr, after having adjusted the pH to 7.0, was loaded downflow on a strong cationic exchange chromatographic column in order to bind, and consequently concentrate, all the basic proteins and peptides present in the solution. The bound fraction (SNprb) was eluted with the upflow direction and then diafiltered through a phosphate buffer 50 mM pH 7.0 and concentrated by means of a tangential flow system through a filter having a cut-off of 3 KDa. The intermediate mixture obtained by this procedure was hereinafter called BASIC FRACTION.
(v.SN.5) The unbound fraction (SNprub) of the step (v.SN.4), after having adjusted the pH to 7.0, was then loaded on a strong anionic exchange resin in order to bind, and consequently concentrate, all the acidic proteins and peptides present in the solution. The bound fraction was eluted with the upflow direction and then diafiltered through a phosphate buffer 50 mM pH 7.0 and concentrated by means of a tangential flow system through a filter having a cut-off of 3 KDa. The intermediate obtained by this procedure is hereinafter called ACIDIC FRACTION.
(v.SN.6) Both the BASIC and ACIDIC FRACTIONS were independently fractionated by molecular weight through a series of diafiltrations and concentrations of the related permeates. The fractions were characterized by the following molecular weight ranges:
   More than 300 KDa
   Between 100 and 300 KDa
   Between 50 and 100 KDa
   Between 30 and 50 KDa
   Between 10 and 30 KDa
   Between 5 and 10 KDa
   Between 1 and 5 KDa
(v.SN.7) Both the BASIC and ACIDIC FRACTIONS from the step (v.SN.6) were further fractionated by molecular weight through a gel permeation chromatography.
(v.SN.8) The fraction SNr was further homogenized and fractionated following the steps from (v.SN.3) to (v.SN.7). This way the alkaline phosphatase and the lysozyme were obtained with the following yield: 38.840 DEA Units of alkaline phosphatase activity per kg of mucosa (one DEA unit will hydrolyse 1 µmol of p-nitrophenyl phosphate per minute at pH 9.8 at 37°C, as known by the skilled person), and 1.014.983 U-FIP of lysozyme activity per kg of mucosa (one FIP unit will produce a change in A₄₅₀ nm of 0.001 per minute at pH 6.24 at 25 °C, using a suspension of Micrococcus lysodeikticus as substrate, in a 2.6 ml reaction mixture, as known by the skilled person).

Alternatively, both BASIC and ACIDIC FRACTIONS could have been fractionated by salting out methods, such as through the addition of ammonium sulphate or by the addition of an organic solvent such as acetone.

Alternatively, the BASIC and ACIDIC FRACTIONS could have been loaded on weak cationic and anionic exchange column, respectively, and the single proteins and peptides, can be eluted by means of ionic strength or pH gradient.

This example provides evidence of the capability of the process to simultaneously obtain with proper amounts and purity grade the one or more enzymes and heparin from the mammalian intestine mucosa. As example with the described process an enzymatic activity of 38.840 DEA Units of alkaline phosphatase activity per kg of mucosa, and 1.014.983 U-FIP of lysozyme activity per kg of mucosa are obtained, while heparin, a compound usually obtained from this tissue is also obtained with 0,047 MIU of heparin activity per Kg of mucosa. These heparin values are higher than the ones obtained with the standard extraction process of the prior art. Data have been exemplified with alkaline phosphatase and lysozyme from SNr, but the different steps, namely (v.SN.4) and (v.SN.5) show also how to separate and to obtain all the proteins according to their isoelectric point, by choosing the appropriate pH and pH gradient. Other method steps, in particular those based on the molecular weight of the proteins were also applied (see v.SN.6 and v.SN.7).

Indeed, the proposed method for the simultaneous obtention of heparin, proteins and peptides in native state by fractionation of mammalian intestine mucosa is based on the properties of the proteins contained in the mucosa, mainly on the isoelectric point at a predetermined pH, and/or their molecular weight, and/or solubility in water or organic solvents.

### Example 3a. Industrial process (II) for obtaining heparin and by-side products (i.e., native proteins and peptides) from mammalian intestine mucosa

The method included the following steps:
(i) An aqueous extract of a mammalian intestinal mucosa was obtained (i.e., separating the mucosa of the intestine with standard methods and dissolving/suspending it in water or in an aqueous buffer); and then to said aqueous extract of mammalian intestinal mucosa (hereinafter called MUCOSA) sodium metabisulfite was added up to a concentration of 2%. Equivalent substances can be chosen as a preservative and antioxidant agent;
(ii) MUCOSA was further diluted with 2 volumes of deionized water. The output of this procedure yielded an intermediate hereinafter called DILUTED MUCOSA. Alternatively, the diluted mucosa could have been obtained by diluting the MUCOSA with an aqueous solution, comprising one of the following detergents Tween 20, Tween 80, Triton X-100 or X-114, deoxycholic acid, Brij 35 or 58, BigCHAP or deoxy BigCHAP, MEGA 8, MEGA 9 or MEGA 10, Octyl beta-glucoside;
(iii) DILUTED MUCOSA was submitted to homogenization by a rotor-stator homogenizer at the G-force of 1700 g, during 2 to 4 minutes at a temperature between 2°C and 8°C to lyse the cell membranes and to obtain a stable homogenate of the mucosa comprising heparin and mucosa proteins and peptides (herein after called HOMOGENATE). Alternative homogenization modes can be performed by other techniques selected from the group consisting of mechanical or physical cell lysis of mammalian intestinal mucosa by mechanical disruption, shear fluid forces, high pressure or cavitation. Homogenization can be performed in the presence of detergents;
(iv) Heparin and the mucosa proteins and peptides contained in the HOMOGENATE obtained in step (iiia) were separated by centrifugation in order to obtain liquid and solid phases, each comprising different proportion of one or more of heparin, and other mucosa proteins and peptides. The centrifugation was carried out at a G-force of 3300 g for 30 minutes and at a temperature below 37°C (preferably between 0 and 25°C) to obtain: a solid phase containing heparin, (hereinafter called PELLET or P), and a liquid phase containing a mixture of mucosa proteins and peptides (hereinafter called SUPERNATANT or SN). Alternatively, the separation of the PELLET and the SUPERNATANT can be achieved by tangential microfiltration through 0.2 µm membrane. The SN contains, among other proteins and peptides, glucagon like peptide-1 (GLP-1), thymosin beta-4, vaso intestinal peptide, lysozyme, lactotransferrin, regenerating islet-derived protein 3 (Reg III), regenerating islet-derived protein IV (Reg IV), matrix metalloproteinase-9, 72 kDa type IV collagenase, interstitial collagenase, bactericidal permeability-increasing protein, pulmonary surfactant-associated protein D, antibacterial protein PR-39, angiogenin, alkaline phosphatase, mucin-2, trypsin, aminopeptidases, carboxypeptidases, catalase, triacylglycerol lipase, phospholipases, acid sphingomyelinase-like phosphodiesterase, alpha-amylase, alpha-galactosidase, sucrase-isomaltase, peroxiredoxin-6, superoxide dismutase, apolipoprotein A1, annexin-5, galectin-1, diazepam binding inhibitor, immunoglobulins, gastrotropin, hemoglobin, as confirmed by proteomic analysis. The SN also contains phytase from exogenous origin, as confirmed by enzymatic assay.
(v.P) The PELLET was then submitted to the standard extraction method to obtain and purify heparin for pharmaceutical applications (This step can also be labelled as (iv.a), as previously indicated). In brief, the pellet was treated with an enzymatic alkaline proteolysis at 58°C and pH 7.7-8 during 2 hours. After the hydrolysis, pH was decreased to 6.6 and temperature was increased to 90°C and maintained during 15 minutes to precipitate the fat. The product was centrifuged at a G-force of 3134 g for 15 minutes, and the supernatant was decantated and filtrated through a 300 µm filter to separate the fat fraction. Then, an anion exchange resin was added to the supernatant in a proportion between 0,1 and 1% w/w (preferably 0,2%) to capture the heparin. The mixture was kept under stirring overnight, and resin was separated from the liquid with a mesh filter, and rinsed with osmotized water. Proteins were eluted from the resin with three successive additions of NaCl 3.5% (w/v) at 45°C for 30 minutes each. Finally, heparin was eluted with three successive additions of NaCl 18%, 20%, and 20% at 45°C for 30 minutes each. The 3 heparin eluates were combined and analyzed yielding an average of 0,047 MIU of heparin activity per Kg of mucosa (one unit of heparin is the quantity of heparin required to keep 1 ml of cat's blood fluid for 24 hours at 0°C; it is equivalent approximately to 0.002 mg of pure heparin, as known by the skilled person).
(v.SN) (or (v.b') as previously indicated) The SUPERNATANT was submitted to successive filtration, ultrafiltration steps, protein precipitation and/or gel permeation and ion exchange chromatography steps in order to separate the proteins and peptides according to their solubility, isoelectric point and molecular weight (see particular steps (iv.SN.1) to (iv.SN.7) as follows).
(v.SN.1) SUPERNATANT was gross filtered through a 100 µm cut-off filter in order to retain small particles and lipid residues which are lighter and, consequently, not pelleted. The intermediate obtained by this procedure was hereinafter called FILTERED SUPERNATANT (SNf)
(v.SN.2) The FILTERED SUPERNATANT (SNf) was optionally diafiltered by means of a tangential flow filtration equipment through a filter having a cut-off of less than 0.1 µm. The diafiltration was carried out by substituting the permeate with at least 4 volumes of a buffer, preferably between 5 and 7 volumes. The aim of this step was to recover both the retentate (SNr) and the permeate (SNp).
(v.SN.3) The permeate (SNp) was further diafiltered by means of a tangential flow filtration equipment through a filter having a cut-off less 3 kDa in order to concentrate it (SNpr).
(v.SN.4) The fraction SNpr (or the fraction SNf), after having adjusted the pH to 7.0, was loaded downflow on a strong anionic exchange chromatographic column in order to bind, and consequently concentrate, all the acidic proteins and peptides present in the solution. The bound fraction (SNprb) was eluted with the upflow direction and then diafiltered through a phosphate buffer 50 mM pH 7.0 and concentrated by means of a tangential flow system through a filter having a cut-off of 3 KDa. Alternatively, the anionic exchange chromatography can be done in batch, adding between 0,5 and 1,5% (w/w) (preferably 1%) of strong anionic exchange resin to the fraction. The intermediate mixture obtained by this procedure was hereinafter called ACIDIC FRACTION (AF). The ACIDIC FRACTION (AF) contains, among other, the proteins and peptides identified by the proteomic analysis (shown in example 3b).
(v.SN.5) The unbound fraction (SNprub) of the step (v.SN.4), after having adjusted the pH to 7.0, was then loaded on a strong cationic exchange column in order to bind, and consequently concentrate, all the basic proteins and peptides present in the solution. The bound fraction was eluted with the upflow direction and then diafiltered through a phosphate buffer 50 mM pH 7.0 and concentrated by means of a tangential flow system through a filter having a cut-off of 3 KDa. Alternatively, the cationic exchange chromatography can be done in batch, adding between 5 and 15% (w/w) (preferably 10%) of strong cationic exchange resin to the fraction. The intermediate obtained by this procedure is hereinafter called BASIC FRACTION (BF). The BASIC FRACTION (BF) contains, among other, the proteins and peptides identified by the proteomic analysis (shown in example 2b). Two subfractions were eluted from the BASIC FRACTION using different NaCl concentrations. By washing the resin with 2% NaCl, a subfraction (BF2) was eluted and characterized by enzymatic assay and proteomic analysis. It contained, among other proteins, lysozyme with an activity of 150 U-FIP/mL, lactotransferrin, phosphoinositide phospholipase C, phospholipase D, antibacterial protein PR-39, regenerating family member 4 and angiogenin. By washing the resin with 7% NaCl another subfraction (BF7) was eluted containing the rest of the proteins from the BASIC FRACTION. Both subfractions were tested for antimicrobial activity as detailed in Example 2c.
(v.SN.6) Both the BASIC and ACIDIC FRACTIONS or their subfractions were independently fractionated by molecular weight through a series of diafiltrations and concentrations of the related permeates. The fractions were characterized by the following molecular weight ranges:
   More than 300 KDa
   Between 100 and 300 KDa
   Between 50 and 100 KDa
   Between 30 and 50 KDa
   Between 10 and 30 KDa
   Between 5 and 10 KDa
   Between 1 and 5 KDa
(v.SN.7) Both the BASIC and ACIDIC FRACTIONS from the step (v.SN.6) were further fractionated by molecular weight through a gel permeation chromatography.
(v.SN.8) The unbound fraction (SNprubub) of the step (v.SN.5) contains, among other, the proteins and peptides identified by the proteomic analysis (shown in example 2b). SNprubub can be used as an ingredient for feed and/or food applications, or it can be further fractionated by means of successive filtration, and ultrafiltration steps, solvent extraction, protein precipitation, and/or gel permeation and affinity chromatography steps. On a specific case, SNprubub was treated with butanol 40% (w/w) to extract alkaline phosphatase and mucin-2 in the aqueous phase. This aqueous phase (SNprububaq) was characterized by enzymatic assay and proteomic analysis. It contains, among other, the proteins and peptides identified by the proteomic analysis (shown in example 2b). In particular, alkaline phosphatase with an activity of 10,7 DEA U/mL, and mucin-2. SNprububaq was tested for antimicrobial activity as described in the example 2c. To further purify its components, SNprububaq phase was then treated with acetone 30% (w/w), and the resulting supernatant was treated with acetone 60% (w/w) to precipitate ALP and separate from MUC2 that remained in the supernatant. ALP and MUC2 were then purified by means of affinity chromatography to obtain pharmaceutical grade proteins suitable for medical applications. Alternatively, precipitation with ammonium sulphate, aluminium, or their combinations with the previous treatments can be used. The supernatant of any of its derived fractions can also be treated previously with phosphatidylinositol-specific phospholipase C to detach alkaline phosphatase and other membrane proteins in order to improve their separation and purification, and obtain higher yields.
(v.SN.8) The fraction SNr was further homogenized and fractionated following the steps from (v.SN.3) to (v.SN.7).

This way the enzymes alkaline phosphatase and lysozyme were obtained with the following yield: 38.840 DEA Units of alkaline phosphatase activity per kg of mucosa (one DEA unit will hydrolyse 1 µmol of p-nitrophenyl phosphate per minute at pH 9.8 at 37°C, as known by the skilled person), and 1.014.983 U-FIP of lysozyme activity per kg of mucosa (one FIP unit will produce a change in A₄₅₀ nm of 0.001 per minute at pH 6.24 at 25 °C, using a suspension of Micrococcus lysodeikticus as substrate, in a 2.6 ml reaction mixture, as known by the skilled person). Mucin was isolated with a significant yield, as measured by 0,16 g of N-acetylneuraminic acid per kg of mucosa.

Alternatively, both BASIC and ACIDIC FRACTIONS could also have been fractionated by salting out methods, such as through the addition of ammonium sulphate or by the addition of an organic solvent such as butanol or acetone.

Alternatively, the BASIC and ACIDIC FRACTIONS could have been loaded on weak cationic and anionic exchange column, respectively, and the single proteins and peptides, can be eluted by means of ionic strength or pH gradient.

This example provides evidence of the capability of the process to simultaneously obtain with proper amounts and purity grade the one or more enzymes and heparin from the mammalian intestine mucosa. As example with the described process an enzymatic activity of 38.840 DEA Units of alkaline phosphatase activity per kg of mucosa, and 1.014.983 U-FIP of lysozyme activity per kg of mucosa are obtained, as well as 0,16 g of N-acetylneuraminic acid per kg of mucosa corresponding to sialylated glycoproteins like mucin-2, while heparin, a compound usually obtained from this tissue is also obtained with 0,047 MIU of heparin activity per Kg of mucosa. These heparin values are higher than the ones obtained with the standard extraction process of the prior art. Data have been exemplified with alkaline phosphatase, mucin-2 and lysozyme from SNr, but the different steps, namely (v.SN.4) and (v.SN.5) show also how to separate and to obtain all the proteins according to their isoelectric point, by choosing the appropriate pH and pH gradient. Other method steps, in particular those based on the molecular weight of the proteins were also applied (see v.SN.6 and v.SN.7).

Indeed, the proposed method for the simultaneous obtention of heparin, proteins and peptides in native state by fractionation of mammalian intestine mucosa is based on the properties of the proteins contained in the mucosa, mainly on the isoelectric point at a predetermined pH, and/or their molecular weight, and/or solubility in water or organic solvents.

### Example 3b. Proteomic study of fractions obtained during the industrial process (II) of example 2a.

### Objective

To identify and annotate the proteins present in 13 freeze-dried fractions of porcine intestinal mucosa and to estimate their relative abundance within each fraction.

### Methods

### Sample processing

- Samples P01-P13
- Sample presentation: Solution
- Digesting Enzyme: Trypsin (0.1 µg/µL, Promega)
- Digestion protocol: Protocol_MS01_InSolTrypDig.doc
- Final peptide concentration / volume: 50 µL (3% ACN 1% FA)

### Sample analysis

### nanoLC conditions

- Injected volume: 5 µL
- Chromatograph: Thermo Scientific Dionex Ultimate 3000
- Trap columna: µ-precolumn 300 µm i.d. × 5 mm PepMap100, 5 µm, 100 Å, C18 (Thermo Scientific)
- Analytical column: NanoEase MZ HSS T3 column (75 µm × 250 mm, 1.8 µm, 100 Å) (Waters)
- Eluents: A. H2O 0.1% formic acid; B. CH3CN 0.1% formic acid
- Gradient: 3% to 35% of B in 60 min + 35% to 50% in 5 min + 50% to 85% in 2 min
- Flow rate: 250 nL/min

### LC-MS coupling

- Source: Advion Triversa Nanomate (Advion BioSciences, Ithaca, NY, USA) nESI through chip technology
- Spray voltage: 1.7 kV
- Delivery pressure: 0.5 psi
- Mode: Positive

### MS conditions

- Mass spectrometer: Orbitrap Fusion Lumos^{™} Tribrid (Thermo Scientific)
- Spray voltage: 1.7 kV
- Ion transfer tube temp.: 275°C
- RF Lens: 30%
- m/z range: 375-1500 a.m.u
- Data dependent mode: DDA with Top Speed (most intense)
- Fragmentation method: HCD
- Collision energy: 38%
- AGC: 1x104
- Detection: Orbitrap (MS1 120 k), Orbitrap (MS2 30 k)
- Instrument acquisition software: Xcalibur software vs 4.2.28.14 (Thermo Scientific)

### Data analysis

### Database search

- Search software #1 / version: MaxQuant [2] / v2.0.2.0
- Search node #1 software #1: Andromeda
- Database: Uniprot Pig released 2022_11
- Enzyme: Trypsin (full) with 2 missed cleavages
- Dynamic modifications: Oxidation in M and P; Acetylation in protein N-terminus
- Static modifications: Carbamidomethylation in C
- Precursor mass tolerance: 10 ppm
- MS/MS mass tolerance: 0.02 Da

iBAQ (Intensity Based Absolute Quantification) values are the raw intensities divided by the number of theoretical peptides. This normalization algorithm is able to estimate protein amounts within each sample. IBAQ intensities [1] are calculated within MaxQuant [2] software.

### Results

Fractions had a varying number of protein groups quantified (from 113 to 1905). Based on the results we can conclude that, among others, the following proteins with potential commercial application are isolated or concentrated in the corresponding fractions:

### Acidic Fraction (AF): Pr-glucagon, Glucagon-like peptide-1, Thymosin beta-4, Vasointestinal peptides

### Basic Fraction (BF): Lysozyme, Lactotrasferrin; 72 kDa type IV collagenase

Interstitial collagenase, Matrix metalloproteinase-9, Regenerating family member III / regenerating islet-derived protein 3 (Reg III), Regenerating family member IV / regenerating islet-derived protein IV (Reg IV), Antibacterial protein PR-39, Angiogenin, Phosphoinositide phospholipase C, Phospholipase D, Pulmonary surfactant-associated protein D

SNprubub: Alkaline phosphatase, Mucin-2, Immunoglobulin M, Immunoglobulin A, Sucrase-Isomaltase, Alpha-amylase, Hemoglobin, Aminopeptidases, Maltase-glucoamylase, Catalase, Carboxypeptidases, Alpha-galactosidase, Peroxiredoxin-6, Superoxide dismutase, Acid sphingomyelinase-like phosphodiesterase, Diazepam binding inhibitor, Gastrotropin, Apolipoprotein-1, Annexin-5, Galectin-1, Triacylglycerol lipase , Phospholipase, Thymosin beta-4

### SNprububaq:

Alkaline phosphatase, Mucin-2, Immunoglobulin M, Immunoglobulin A, Sucrase-Isomaltase, Alpha-amylase, Hemoglobin, Aminopeptidases , Maltase-glucoamylase , Catalase , Carboxypeptidases, Alpha-galactosidase, Peroxiredoxin-6, Superoxide dismutase, Acid sphingomyelinase-like phosphodiesterase, Diazepam binding inhibitor, Gastrotropin, Apolipoprotein-1, Annexin-5, Galectin-1

### Example 3c. Screening of the antimicrobial and antifungal activity of porcine extracts obtained during the industrial process (II) of example 2a against different pathogens

### Objective

Evaluation of the antimicrobial and antifungal activity of 9 fractions of porcine intestine mucosa against 3 human pathogenic bacteria, 1 phytopathogenic bacteria and 1 phytopathogenic fungus.

### Methods

### Pathogens

Antibacterial and antifungal activity were tested against the pathogens in Table 1:

**Table 1:**

| Pathogen | Strain |
|---|---|
| Salmonella typhymurium (St) | ATCC 700720 |
| Staphylococcus aureus (Sa) | CECT 435 |
| Fusarium oxysporum f. sp. Lycopersici (Fo) | ATCC 201829 |

The inoculums were obtained from pure cultures of growing strains, seeded in solid Luria-Bertani (LB) medium in the case of bacteria, and Potato dextrose agar (PDA) medium in the case of the fungus. The human pathogens (St, and Sa) were incubated for 24 h at 37 °C, and the fungus (Fo) for 7 days at 23 °C. All bacterial suspensions were prepared to a final concentration of 2x107 CFU/ml. In the case of the test with the fungus, a suspension of conidia was prepared at a final concentration of 2x104 conidia/ml.

### Treatments

The antimicrobial activity of 4 fractions and two control samples *(see annex slide 5)* was evaluated at a concentration of 0.5X. The antimicrobial effect was compared with the reference antibiotic levofloxacin (50 mg/L) in the case of bacteria, and cycloheximide (50 mg/L) in the case of the fungus. An untreated control was also included where the product was replaced with water (control positive for microbial growth), a negative control with culture medium without product nor microbial suspension (to rule out possible contamination of the environment). Product controls were included, where each product was incubated without microbial suspension or culture medium (to discard product contamination).

### Assay

The antimicrobial activity of the different products against the 5 pathogens was determined by a growth inhibition assay. Bacterial suspensions were prepared with sterile distilled water and adjusted to a stock concentration of 2x107 CFU/ml in liquid LB medium. In the case of the fungus, the conidia suspension was also prepared with sterile distilled water and adjusted to a concentration of 2x104 conidia/ml in the PDA medium. The growth inhibition assay consisted of mixing 100 µl of the product with 100 µl of the bacterial/fungal suspension, obtaining a final volume of 200 µl in each well of the microplate (bacterial suspensions at a final concentration of 1x107 CFU/ml and fungal suspension at a final concentration of 1x104 conidia/ml). The microplates were incubated at 37 °C in the case of the human pathogens (St and Sa). The fungus was incubated for 7 days at 20°C with constant shaking.

The growth kinetics of the different pathogens in the presence of the products was analyzed in triplicate (3 wells per product and pathogen) using automated systems that allow microbial growth to be monitored through absorbance readings at 600 nm (Bioscreen C MBR, Labsystems, Finland and Varioskan flash, Thermo Electron Corporation, USA) every hour in the case of bacteria (for 24 h) and every 30 min in the case of fungus (for 7 days). Microbial growth was quantified by analyzing the area under the growth curve, calculating the percentage of growth inhibition for each pathogen treated with each product.

### Results

The percentage of growth inhibition for each pathogen treated with each product is shown in Table 2 .

**Table 2 The % inhibition value represents the average of the 3 replicates analyzed for each pathogen and product. Ab: Antibiotic control**

| | Pathogens | | |
|---|---|---|---|
| Product | St | Sa | Fo |
| AF | 7,5 | 41,8 | 43,7 |
| BF2 | 0 | 46,8 | 31,3 |
| BF7 | 0 | 41,8 | 0 |
| SNprububaq | 69,8 | 38,6 | 14,4 |
| LYZst | 0,6 | 30 | 37,7 |
| ALPst | 0 | 27,1 | 0 |
| Ab | 99,8 | 97,6 | 87,4 |

| | | | |
|---|---|---|---|
| • All fractions show antimicrobial activity against *Staphylococcus aureus.* BF2 (contains LYZ) has the highest activity (higher than LYZ control) • AF and SNprububaq show activity against *Salmonella thypimurium.* SNprububaq (contains ALP + Mucin) has the highest activity (higher than LYZ control) • AF, BF2 and SNprububaq show activity against *Fusarium oxysporum.* AF has the highest activity (higher than LYZ control) | | | |

The fractions AF, BF2 and SNprububaq show significant antimicrobial potential.

### Citation List

### Patent Literature

- CN107236059
- CN106035980B
- CN102839162B
- RU2610669
- US20140369989

### Non Patent Literature

- Fontaine et al. (1985). Absence d'effet de la vitamina D sur la phytase et la phosphatase alcaline intestinales: relation avec I'absorption du phosphore phytique chez le porc. Reprod.Nutr. Dévelop., 25(4A), 717-727.
- Lackeyram et al. (2010). Early weaning reduces expression of alkaline phosphatase in pigs. The Journal of Nutrition 140, pp.461-468

## Claims

1. A method for the simultaneous obtention of heparin, proteins and peptides in native state by fractionation of mammalian intestine mucosa, comprising the following steps:
(i) adding to an aqueous extract of mammalian intestinal mucosa a preservative and antioxidant agent to obtain a preserved mucosa, or preserving the extract at a temperature allowing preserving proteins, peptides and heparin in native state;
(ii) optionally diluting the preserved mucosa with up to 100 volumes of deionized water, to obtain a diluted mucosa
(iiia) submitting diluted mucosa of step (ii) or preserved mucosa of (i) to homogenization at a temperature of less than 40 °C, optionally in presence of detergents and/or hydrolytic enzymes such as phospholipases, to lyse cell membrane of mucosa cells to obtain a stable homogenate of the mucosa comprising heparin and mucosa proteins and peptides;
(iva) separating heparin and the mucosa proteins and peptides contained in the stable homogenate obtained in step (iiia) by one or more of physical and/or chemical means selected from the group consisting of centrifugation and filtration (microfiltration and/or ultrafiltration), optionally using detergents, and combinations thereof, to obtain a pellet (P) or retentate fraction comprising heparin and proteins; and a supernatant (SN) or permeate fraction comprising mucosa proteins and peptides;
(v.a') submitting the pellet (P) or retentate fraction of (iva) comprising heparin to an alkaline proteolytic process by means of proteolytic enzymes to obtain a mixture of heparin and a protein hydrolysate, submiting the mixture with heparin to one or more precipitation steps, and optionally to one or more of extractive steps, and to one or more of a chromatography to purify the said heparin from the protein hydrolysate; and
(v.b') submitting the supernatant (SN) or permeate of (iva) comprising a mixture of solubilized and/or suspended mucosa proteins and peptides to one or more of successive filtration, ultrafiltration steps, solvent extraction, protein precipitation, enzymatic hydrolysis, gel permeation, ion exchange chromatography, size exclusion chromatography, or affinity chromatography steps, being exchangeable the order or sequence of the steps, in order to separate the proteins and peptides according to any of their solubility, isoelectric point and/or molecular weight.

2. The method according to claim 1, wherein step (iva) of separation is carried out by centrifugation.

3. The method according to any of the claims 1-2, which further comprises:
(v.b.1') submitting the supernatant (SN) or permeate of step (v.b') comprising a mixture of solubilized and/or suspended mucosa proteins and peptides to filtration through an up to 100 µm cut-off filter in order to retain small particles and lipid residues which were not pelleted in step (iva), to obtain a filtered supernatant comprising most of the mucosa proteins and peptides.

4. The method according to claim 3, which further comprises:
(v.b.4') submitting the supernatant of step (v.b.1') to an anionic exchange chromatography column to obtain a bound fraction (SNbf') and an unbound fraction (SNubf'), and eluting the bound fraction and then diafilter and concentrate it by means of a tangential flow system through a filter having a cut-off from 1 to 5 KDa to obtain an acidic fraction of the supernatant comprising acidic proteins and peptides.

5. The method according to claim 4, which further comprises:
(v.b.5') submitting the unbound fraction (SNubf') of step (v.b.4') to a cationic exchange chromatography column to obtain a bound fraction (SNubfbf') and an unbound fraction (SNubub'), and eluting the bound fraction then diafilter and concentrate it by means of a tangential flow system through a filter having a cut-off from 1 to 5 KDa to obtain a basic fraction of the supernatant comprising basic proteins and peptides.

6. The method according to claim 3, which further comprises:
(v.b.4a) submitting the supernatant of step (v.b.1') to a cationic exchange chromatography column to obtain a bound fraction (SNbf) and an unbound fraction (SNubf), and eluting the bound fraction and then diafilter and concentrate it by means of a tangential flow system through a filter having a cut-off from 1 to 5 KDa, to obtain a basic fraction of the supernatant comprising basic proteins and peptides.

7. The method according to claim 6, which further comprises:
(v.b.5a) submitting the unbound fraction (SNubf) of step (v.b.4a) to an anionic exchange chromatography column to obtain a bound fraction (SNubfbf) and an unbound fraction (SNubub), and eluting the bound fraction and then diafilter and concentrate it by means of a tangential flow system through a filter having a cut-off from 1 to 5 KDa, to obtain an acidic fraction of the supernatant comprising acidic proteins and peptides.

8. The method according to any of the claims 4-7, wherein any of the basic or acidic fractions are further submitted to fractionation by means of diafiltration, salting out methods, protein precipitation, solvent extraction, cationic and anionic exchange chromatography, size exclusion chromatography, affinity chromatography and combinations thereof.

9. The method according to any of the claims 1-8, wherein in step (ii) the mucosa is diluted with deionized water comprising one or more detergents.

10. The method according to any of the claims 1-9, wherein homogenization step (iiia) is carried out by a technique selected from the group consisting of mechanical or physical cell lysis of mammalian intestinal mucosa by mechanical disruption, shear fluid forces, high pressure or cavitation.

11. The method according to claim 10, wherein homogenization step (iiia) is carried out by mechanical disruption of the mucosa cells by means of a rotor-stator homogenizer at the g-force of at least 180 g, preferably 1700 g, for a minimum of 30 seconds, preferably between 2 to 4 minutes.

12. The method according to any of the claims 1-9, wherein homogenization step (iiia) is carried out by chemical means including the addition of a detergent to the diluted mucosa of step (ii).

13. A method for the obtention of heparin by fractionation of mammalian intestine mucosa, comprising the steps (i), (ii), (iiia), (iva), and (v.a') as defined in claim 1.

14. A method for the obtention of proteins and peptides in native state by fractionation of mammalian intestine mucosa, comprising the steps (i), (ii), (iiia), (iva), and (v.b') as defined in claim 1.

## Patentansprüche

1. Ein Verfahren zur gleichzeitigen Gewinnung von Heparin, Proteinen und Peptiden im nativen Zustand durch Fraktionierung der Darmschleimhaut von Säugetieren, umfassend die folgenden Schritte:
(i) Zugeben eines Konservierungs- und Antioxidationsmittels zu einem wässrigen Extrakt der Darmschleimhaut von Säugetieren, um eine konservierte Schleimhaut zu erhalten, oder Konservieren des Extrakts bei einer Temperatur, die das Konservieren von Proteinen, Peptiden und Heparin in nativem Zustand ermöglicht;
(ii) wahlweise, Verdünnen der konservierten Schleimhaut mit bis zu 100 Volumina entionisiertem Wasser, um eine verdünnte Schleimhaut zu erhalten;
(iiia) Unterziehen der verdünnten Schleimhaut von Schritt (ii) oder der konservierten Schleimhaut von (i) einer Homogenisierung bei einer Temperatur von weniger als 40 °C, gegebenenfalls in Gegenwart von Detergenzien und/oder hydrolytischen Enzymen wie Phospholipasen, um die Zellmembran von Schleimhautzellen zu lysieren, um ein stabiles Homogenat der Schleimhaut zu erhalten, das Heparin und Schleimhautproteine und -peptide umfasst;
(iva) Trennen von Heparin und den Schleimhautproteinen und -peptiden, die in dem in Schritt (iiia) erhaltenen stabilen Homogenat enthalten sind, durch ein oder mehrere physikalische und/oder chemische Mittel, die aus der Gruppe ausgewählt sind, die aus Zentrifugation und Filtration (Mikrofiltration und/oder Ultrafiltration) besteht, gegebenenfalls unter Verwendung von Detergenzien und Kombinationen davon, um eine Pellet- (P) oder Retentatfraktion, die Heparin und Proteine umfasst zu erhalten; und eine Überstand- (SN) oder Permeatfraktion zu erhalten, die Schleimhautproteine und -peptide umfasst;
(v.a') Unterziehen der Pellet- (P) oder Retentatfraktion von (iva), welche Heparin umfasst, einem alkalischen proteolytischen Verfahren mit Hilfe von proteolytischen Enzymen, um eine Mischung aus Heparin und einem Proteinhydrolysat zu erhalten, Unterziehen der Mischung mit Heparin einem oder mehreren Fällungsschritten und gegebenenfalls einem oder mehreren von Extraktionsschritten und einer oder mehreren von einer Chromatographie, um das Heparin von dem Proteinhydrolysat zu reinigen; und
(v.b') Unterziehen des Überstands (SN) oder Permeats von (iva), der eine Mischung von solubilisierten und/oder suspendierten Schleimhautproteinen und -peptiden umfasst, einem oder mehreren von aufeinanderfolgenden Filtrationsschritten, Ultrafiltrationsschritten, Lösungsmittelextraktion, Proteinfällung, enzymatischer Hydrolyse, Gelpermeation, lonenaustauschchromatographie, Größenausschlusschromatographie oder Affinitätschromatographieschritten, wobei die Reihenfolge oder Sequenz der Schritte austauschbar ist, um die Proteine und Peptide nach ihrer Löslichkeit, ihrem isoelektrischen Punkt und/oder Molekulargewicht zu trennen.

2. Das Verfahren nach Anspruch 1, wobei der Schritt (iva) der Trennung durch Zentrifugation durchgeführt wird.

3. Das Verfahren nach einem der Ansprüche 1-2, das ferner Folgendes umfasst: (v.b.1') Unterziehen des Überstands (SN) oder Permeats von Schritt (v.b'), der eine Mischung aus solubilisierten und/oder suspendierten Schleimhautproteinen und - peptiden umfasst, einer Filtration durch einen Cut-Off-Filter von bis zu 100 µm, um kleine Partikel und Lipidrückstände, welche in Schritt (iva) nicht pelletiert wurden, zurückzuhalten, um einen gefilterten Überstand zu erhalten, der die meisten Schleimhautproteine und -peptide umfasst.

4. Das Verfahren nach Anspruch 3, das ferner Folgendes umfasst:
(v.b.4') Unterziehen des Überstands aus Schritt (v.b.1') einer Anionenaustauschchromatographiesäule, um eine gebundene Fraktion (SNbf ') und eine ungebundene Fraktion (SNubf') zu erhalten, und Eluieren der gebundenen Fraktion und dann Diafiltrieren und Konzentrieren derselben mittels eines Tangentialflusssystems durch einen Filter mit einem Cut-off von 1 bis 5 KDa, um eine saure Fraktion des Überstands zu erhalten, die saure Proteine und Peptide umfasst.

5. Das Verfahren nach Anspruch 4, das ferner Folgendes umfasst:
(v.b.5') Unterziehen der ungebundenen Fraktion (SNubf') von Schritt (v.b.4') einer Kationenaustauschchromatographiesäule, um eine gebundene Fraktion (SNubfbf') und eine ungebundene Fraktion (SNubub') zu erhalten, und Eluieren der gebundenen Fraktion dann Diafiltrieren und Konzentrieren derselben mittels eines Tangentialflusssystems durch einen Filter mit einem Cut-off von 1 bis 5 KDa, um eine basische Fraktion des Überstands zu erhalten, die basische Proteine und Peptide umfasst.

6. Das Verfahren nach Anspruch 3, das ferner Folgendes umfasst:
(v.b.4a) Unterziehen des Überstands aus Schritt (v.b.1') einer Kationenaustauschchromatographiesäule, um eine gebundene Fraktion (SNbf) und eine ungebundene Fraktion (SNubf) zu erhalten, und Eluieren der gebundenen Fraktion und dann Diafiltrieren und Konzentrieren derselben mittels eines Tangentialflusssystems durch einen Filter mit einem Cut-off von 1 bis 5 KDa, um eine basische Fraktion des Überstands zu erhalten, die basische Proteine und Peptide umfasst.

7. Das Verfahren nach Anspruch 6, das ferner Folgendes umfasst:
(v.b.5a) Unterziehen der ungebundenen Fraktion (SNubf) von Schritt (v.b.4a) einer Anionenaustauschchromatographiesäule, um eine gebundene Fraktion (SNubfbf) und eine ungebundene Fraktion (SNubub) zu erhalten, und Eluieren der gebundenen Fraktion und dann Diafiltrieren und Konzentrieren derselben mittels eines Tangentialflusssystems durch einen Filter mit einem Cut-off von 1 bis 5 KDa, um eine saure Fraktion des Überstands zu erhalten, die saure Proteine und Peptide umfasst.

8. Das Verfahren nach einem der Ansprüche 4 bis 7, wobei eine der basischen oder sauren Fraktionen ferner einer Fraktionierung mittels Diafiltration, Aussalzungsverfahren, Proteinfällung, Lösungsmittelextraktion, Kationen- und Anionenaustauschchromatographie, Größenausschlusschromatographie, Affinitätschromatographie und Kombinationen davon unterzogen wird.

9. Das Verfahren nach einem der Ansprüche 1 bis 8, wobei in Schritt (ii) die Schleimhaut mit entionisiertem Wasser verdünnt wird, das ein oder mehrere Detergenzien umfasst.

10. Das Verfahren nach einem der Ansprüche 1 bis 9, wobei der Homogenisierungsschritt (iiia) durch eine Technik durchgeführt wird, die aus der Gruppe ausgewählt ist, die aus mechanischer oder physikalischer Zelllyse der Darmschleimhaut von Säugetieren durch mechanisches Aufbrechen, Scherfluidkräfte, Hochdruck oder Kavitation besteht.

11. Das Verfahren nach Anspruch 10, wobei der Homogenisierungsschritt (iiia) durch mechanisches Aufbrechen der Schleimhautzellen mittels eines Rotor-Stator-Homogenisators bei der g-Kraft von mindestens 180 g, vorzugsweise 1700 g, für mindestens 30 Sekunden, vorzugsweise zwischen 2 bis 4 Minuten, durchgeführt wird.

12. Das Verfahren nach einem der Ansprüche 1 bis 9, wobei der Homogenisierungsschritt (iiia) durch chemische Mittel, einschließlich der Zugabe eines Detergens zu der verdünnten Schleimhaut von Schritt (ii), durchgeführt wird.

13. Ein Verfahren zur Gewinnung von Heparin durch Fraktionierung von Darmschleimhaut von Säugetieren, umfassend die Schritte (i), (ii), (iiia), (iva) und (v.a') wie in Anspruch 1 definiert.

14. Ein Verfahren zur Gewinnung von Proteinen und Peptiden im nativen Zustand durch Fraktionierung der Darmschleimhaut von Säugetieren, umfassend die Schritte (i), (ii), (iiia), (iva) und (v.b') wie in Anspruch 1 definiert.

## Revendications

1. Un procédé d'obtention simultanée d'héparine, de protéines et de peptides à l'état natif par fractionnement de la muqueuse intestinale de mammifère, comprenant les étapes suivantes :
(i) ajouter à un extrait aqueux de muqueuse intestinale de mammifère un agent conservateur et antioxydant pour obtenir une muqueuse préservée, ou conserver l'extrait à une température permettant de conserver les protéines, les peptides et l'héparine à l'état natif ;
(ii) éventuellement diluer la muqueuse préservée avec jusqu'à 100 volumes d'eau désionisée, pour obtenir une muqueuse diluée ;
(iiia) soumettre la muqueuse diluée de l'étape (ii) ou la muqueuse préservée de (i) à une homogénéisation à une température inférieure à 40 °C, éventuellement en présence de détergents et/ou d'enzymes hydrolytiques telles que les phospholipases, pour lyser la membrane cellulaire des cellules de la muqueuse afin d'obtenir un homogénat stable de la muqueuse comprenant de l'héparine et des protéines et peptides de la muqueuse ;
(iva) séparer l'héparine et les protéines et peptides de la muqueuse contenus dans l'homogénat stable obtenu à l'étape (iiia) par un ou plusieurs moyens physiques et/ou chimiques choisis dans le groupe constitué par la centrifugation et la filtration (microfiltration et/ou ultrafiltration), éventuellement à l'aide de détergents, et des combinaisons de ceux-ci, pour obtenir une fraction de granulé (P) ou de rétentat comprenant de l'héparine et des protéines ; et une fraction de surnageant (SN) ou de perméat comprenant des protéines et des peptides de la muqueuse ;
(v.a') soumettre la fraction de granulé (P) ou de rétentat de (iva) comprenant de l'héparine à un processus protéolytique alcalin au moyen d'enzymes protéolytiques pour obtenir un mélange d'héparine et d'un hydrolysat de protéine, soumettre le mélange avec de l'héparine à une ou plusieurs étapes de précipitation, et éventuellement à une ou plusieurs étapes d'extraction, et à une ou plusieurs parmi une chromatographie pour purifier ladite héparine de l'hydrolysat de protéine ; et
(v.b') soumettre le surnageant (SN) ou le perméat de (iva) comprenant un mélange de protéines et de peptides de la muqueuse solubilisés et/ou en suspension à une ou plusieurs étapes successives de filtration, d'ultrafiltration, d'extraction de solvant, de précipitation de protéines, d'hydrolyse enzymatique, de perméation de gel, de chromatographie d'échange d'ions, de chromatographie d'exclusion de taille ou de chromatographie d'affinité, étant échangeable l'ordre ou la séquence des étapes, afin de séparer les protéines et les peptides en fonction de leur solubilité, de leur point isoélectrique et/ou de leur poids moléculaire.

2. Le procédé selon la revendication 1, dans lequel l'étape (iva) de séparation est réalisée par centrifugation.

3. Le procédé selon l'une quelconque des revendications 1 à 2, qui comprend en outre :
(v.b.1') soumettre le surnageant (SN) ou le perméat de l'étape (v.b') comprenant un mélange de protéines et de peptides de la muqueuse solubilisés et/ou en suspension à une filtration à travers un filtre avec un seuil de coupure de jusqu'à 100 µm afin de retenir les petites particules et les résidus lipidiques qui n'ont pas été granulés à l'étape (iva), pour obtenir un surnageant filtré comprenant la plupart des protéines et des peptides de la muqueuse.

4. Le procédé selon la revendication 3, qui comprend en outre :
(v.b.4') soumettre le surnageant de l'étape (v.b.1') à une colonne de chromatographie d'échange anionique pour obtenir une fraction liée (SNbf') et une fraction non liée (SNubf'), et éluer la fraction liée, puis diafiltrer et la concentrer au moyen d'un système d'écoulement tangentiel à travers un filtre ayant un seuil de coupure de 1 à 5 KDa pour obtenir une fraction acide du surnageant comprenant des protéines et des peptides acides.

5. Le procédé selon la revendication 4, qui comprend en outre :
(v.b.5') soumettre la fraction non liée (SNubf') de l'étape (v.b.4') à une colonne de chromatographie d'échange cationique pour obtenir une fraction liée (SNubfbf') et une fraction non liée (SNubub'), et éluer la fraction liée puis diafiltrer et la concentrer au moyen d'un système d'écoulement tangentiel à travers un filtre ayant un seuil de coupure de 1 à 5 KDa pour obtenir une fraction basique du surnageant comprenant des protéines et des peptides basiques.

6. Le procédé selon la revendication 3, qui comprend en outre :
(v.b.4a) soumettre le surnageant de l'étape (v.b.1') à une colonne de chromatographie d'échange cationique pour obtenir une fraction liée (SNbf) et une fraction non liée (SNubf), et éluer la fraction liée puis diafiltrer et la concentrer au moyen d'un système d'écoulement tangentiel à travers un filtre ayant un seuil de coupure de 1 à 5 KDa, pour obtenir une fraction basique du surnageant comprenant des protéines et des peptides basiques.

7. Le procédé selon la revendication 6, qui comprend en outre :
(v.b.5a) soumettre la fraction non liée (SNubf) de l'étape (v.b.4a) à une colonne de chromatographie d'échange anionique pour obtenir une fraction liée (SNubfbf) et une fraction non liée (SNubub), et éluer la fraction liée, puis diafiltrer et la concentrer au moyen d'un système d'écoulement tangentiel à travers un filtre ayant un seuil de coupure de 1 à 5 KDa, pour obtenir une fraction acide du surnageant comprenant des protéines et des peptides acides.

8. Le procédé selon l'une quelconque des revendications 4 à 7, dans lequel l'une quelconque des fractions basique ou acide est en outre soumise à un fractionnement au moyen d'une diafiltration, de procédés de relargage, d'une précipitation de protéines, d'une extraction par solvant, d'une chromatographie d'échange cationique et anionique, d'une chromatographie d'exclusion de taille, d'une chromatographie d'affinité et de combinaisons de celles-ci.

9. Le procédé selon l'une quelconque des revendications 1 à 8, dans lequel dans l'étape (ii), la muqueuse est diluée avec de l'eau désionisée comprenant un ou plusieurs détergents.

10. Le procédé selon l'une quelconque des revendications 1 à 9, dans lequel l'étape d'homogénéisation (iiia) est réalisée par une technique choisie dans le groupe constitué par la lyse cellulaire mécanique ou physique de la muqueuse intestinale de mammifère par rupture mécanique, forces de fluide de cisaillement, haute pression ou cavitation.

11. Le procédé selon la revendication 10, dans lequel l'étape d'homogénéisation (iiia) est réalisée par rupture mécanique des cellules de la muqueuse au moyen d'un homogénéisateur rotor-stator à la force g d'au moins 180 g, de préférence 1700 g, pendant un minimum de 30 secondes, de préférence entre 2 et 4 minutes.

12. Le procédé selon l'une quelconque des revendications 1 à 9, dans lequel l'étape d'homogénéisation (iiia) est réalisée par des moyens chimiques comprenant l'addition d'un détergent à la muqueuse diluée de l'étape (ii).

13. Un procédé pour l'obtention d'héparine par fractionnement de la muqueuse intestinale de mammifère, comprenant les étapes (i), (ii), (iiia), (iva) et (v.a') telles que définies dans la revendication 1.

14. Un procédé pour l'obtention de protéines et de peptides à l'état natif par fractionnement de la muqueuse intestinale de mammifère, comprenant les étapes (i), (ii), (iiia), (iva) et (v.b') telles que définies dans la revendication 1.
